(19) Europäisches Patentamt
European Patent Office
Office européen des brevets

(11) **EP 4 147 703 A1**

(12) **EUROPEAN PATENT APPLICATION**
published in accordance with Art. 153(4) EPC


(43) Date of publication:
**15.03.2023 Bulletin 2023/11**

(21) Application number: **21808649.4**

(22) Date of filing: **19.05.2021**

(51) International Patent Classification (IPC):
***A61K 33/40** (2006.01)* ***A61K 31/14** (2006.01)*
***A61P 35/00** (2006.01)* ***A61P 43/00** (2006.01)*

(52) Cooperative Patent Classification (CPC):
**A61K 31/14; A61K 33/40; A61P 35/00; A61P 43/00**

(86) International application number:
**PCT/JP2021/019079**

(87) International publication number:
**WO 2021/235506 (25.11.2021 Gazette 2021/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **21.05.2020 JP 2020089253**



(71) Applicant: **OSAKA UNIVERSITY**
**Suita-shi**
**Osaka 565-0871 (JP)**

(72) Inventors:
- **TSUJIKAWA, Kazutake**
**Suita-shi, Osaka 565-0871 (JP)**
- **TAKAMORI, Kiyoto**
**Tokyo 105-0021 (JP)**
- **SHIBATA, Takekatsu**
**Tokyo 105-0021 (JP)**

(74) Representative: **J A Kemp LLP**
**80 Turnmill Street**
**London EC1M 5QU (GB)**


(54) **EPITHELIAL CANCER THERAPEUTIC AGENT**


(57) The present invention provides an epithelial cancer therapeutic agent with less side effects. In order to achieve the above object, an epithelial cancer therapeutic agent of the present invention includes: at least one material selected from the group consisting of oxo acid, oxo acid ion, and oxo acid salt, wherein the epithelial cancer therapeutic agent treats or prevents epithelial cancer.


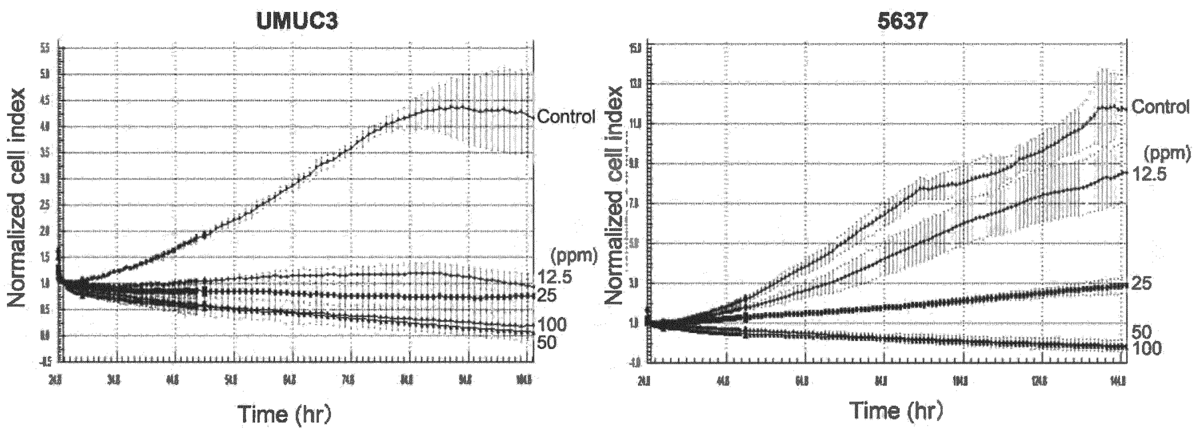


FIG. 1




Processed by Luminess, 75001 PARIS (FR)

## Description

TITLE: TECHNICAL FIELD

The present invention relates to an epithelial cancer therapeutic agent.

BACKGROUND ART

As an epithelial cancer therapeutic agent, a so-called anticancer agent is widely used (Non-Patent Literature 1 and the like). As a bladder cancer therapeutic agent, a so-called BCG is also used.

Citation List

Non-Patent Literature

Non-Patent Literature 1: Hirofumi KOGA, Akito YAMAGUCHI, HINYOKI KEA Urological nursing, 19, 7, 731-736, 2014

SUMMARY OF INVENTION

Technical Problem

However, in general, anticancer agents and BCG have strong side effects, and the patients suffer greatly from the side effects.

With the foregoing in mind, it is an objective of the present invention to provide an epithelial cancer therapeutic agent with less side effects.

Solution to Problem

In order to achieve the above objective, the present invention provides an epithelial cancer therapeutic agent, including: at least one selected from the group consisting of oxo acid, oxo acid ion, and oxo acid salt, wherein the epithelial cancer therapeutic agent is used for treatment or prevention of epithelial cancer. In the present invention, the "treatment" includes "prevention" unless otherwise specified. In the present invention, the "prevention" includes, for example, prevention of recurrence such as prevention of recurrence of epithelial cancer. Advantageous Effects of Invention

According to the present invention, it is possible to provide an epithelial cancer therapeutic agent with less side effects.

BRIEF DESCRIPTION OF DRAWINGS

[FIG. 1] FIG. 1 shows graphs showing the results of the proliferation assays for UM-UC-3 and 5637 cells performed using MA-T.

[FIG. 2] FIG. 2 shows photographs showing the results of the time-lapse photography of bladder cancer cells immediately after the start of photographing after MA-T being added or not added (0-hour culture).

[FIG. 3] FIG. 3 shows photographs showing the results of the time-lapse photography of bladder cancer cells at 48 hours after the start of photographing after being added or not added with MA-T (48-hour culture).

[FIG. 4] FIG. 4 shows the results of the apoptotic analysis of 5637 cells to which MA-T was added and 5637 cells to which no MA-T was added.

[FIG. 5] FIG. 5 is a photograph showing reduction of bladder tumors due to intravesical administration of MA-T, taken by *in vivo* imaging of mice.

[FIG. 6] FIG. 6 is a graph of body weight changes in MA-T intravesically administered mice.

[FIG. 7] FIG. 7 shows photomicrographs showing the results of the histopathological analysis of the bladders of the mice of FIG. 6.

[FIG. 8] FIG. 8 shows graphs showing luminescence and inhibition of increase in bladder weight due to the anti-tumor effect of MA-T by transplantation of luciferase-expressing bladder cancer cells into mice, and also shows photographs showing the results of histopathology analysis of the tumor.

[FIG. 9] FIG. 9 shows a graph showing the time-dependent changes in inhibition of increase in tumor tissue volume and a graph showing the inhibition of increase in tumor weight by transplantation into mice, of a postoperative specimen of MA-T treated bladder cancer .

[FIG. 10] FIG. 10 shows photographs showing the results of the tumor histopathology analysis of FIG. 9.

[FIG. 11] FIG. 11 shows a graph (left graph) showing the time when a 500 ppm $NaClO_2$ aqueous solution was contacted with UM-UC-3 cells and a graph (right graph) showing the concentration-dependency of a $NaClO_2$ aqueous solution on cell proliferation of each bladder cancer cell.

[FIG. 12] FIG. 12 shows photomicrographs of UM-UC-3 cells immediately (0 h) after or 24 hours (24-hour-culture) after being incubated with PBS or $NaClO_2$ for 120 minutes and removed.

[FIG. 13] FIG. 13 shows the apoptosis-induction of UM-UC-3 cells contacted with a 500 ppm $NaClO_2$ aqueous solution.

[FIG. 14] FIG. 14 shows graphs showing the results of the cell cycle analysis of UM-UC-3 cells after 24 hours (overnight) of culture after being contacted with a 500 ppm $NaClO_2$ aqueous solution.

[FIG. 15] FIG. 15 shows the anti-tumor effect of a $NaClO_2$ epithelial cancer therapeutic agent. In the graph on the lower left of FIG. 15, the horizontal axis indicates the number of days from 12 days after tumor transplantation, and the vertical axis indicates the tumor volume ($mm^3$). The photograph on the upper side of FIG. 15 shows a photograph of the bladder dissected and excised on day 27 after the transplantation, and the graph on the lower right of FIG. 15 shows the weight (mg) of the excised tumor.

[FIG. 16] FIG. 16 shows a graph and fluorescent microscopic images demonstrating time-dependent changes in radical production by contacting $NaClO_2$, benzethonium chloride, and MA-T with bladder cancer cells.

[FIG. 17] FIG. 17 shows graphs of viability of tongue cancer cells "HSC-3" and oral carcinoma cells "HO-1-u-1" after 48-hour-culture after being contacted with a 500 ppm $NaClO_2$ aqueous solution for 2 hours.

[FIG. 18] FIG. 18 shows graphs of viability of uterine cancer cells "HeLa" and cervical cancer cells "HeLaS3" after 48-hour-culture after being contacted with a 500 ppm $NaClO_2$ aqueous solution for 2 hours.

[FIG. 19] FIG. 19 shows graphs of viability of colon cancer cells "HT29" and colon cancer cells "HT116" after 48-hour-culture after being contacted with a 500 ppm $NaClO_2$ aqueous solution for 2 hours.

[FIG. 20] FIG. 20 shows graphs showing the effects of the use of a $NaClO_2$ aqueous solution alone and the use of the $NaClO_2$ aqueous solution and benzethonium chloride (radical generating catalyst) in combination, on the proliferation inhibition of uterine cancer cells "HeLa".

[FIG. 21] FIG. 21 shows graphs showing the effects of the use of a $NaClO_2$ aqueous solution alone and the use of the $NaClO_2$ aqueous solution and benzethonium chloride (radical generating catalyst) in combination on the proliferation inhibition of cervical cancer cells "HeLaS3".

[FIG. 22] FIG. 22 shows graphs of the effect of a $NaClO_2$ aqueous solution on the proliferation inhibition of pancreatic cancer cells "PK05" and "PK10".

DESCRIPTION OF EMBODIMENTS

Hereinafter, the present invention will be described in more detail with reference to examples. The present invention, however, is not limited by the following descriptions.

In the present invention, when a compound (e.g., ammonium or the like to be described below) has an isomer such as a tautomer or a stereoisomer (e.g., a geometric isomer, a conformer, and an optical isomer), any isomer can be used unless otherwise specified. In addition, in the present invention, when a substance (for example, an oxo acid, an oxo acid ion, a radical generating catalyst, and the like to be described below) can form a salt, the salt can also be used unless otherwise specified. The salt may be an acid addition salt or a base addition salt. Furthermore, the acid forming the acid addition salt may be an inorganic acid or an organic acid, and the base forming the base addition salt may be an inorganic base or an organic base. Examples of the inorganic acid include, but are not limited to, sulfuric acid, phosphoric acid, hydrofluoric acid, hydrochloric acid, hydrobromic acid, hydriodic acid, hypofluorous acid, hypochlorous acid, hypobromous acid, hypoiodous acid, fluorine acid, chlorous acid, bromous acid, iodous acid, fluoric acid, chloric acid, bromic acid, iodic acid, perfluoric acid, perchloric acid, perbromic acid, and periodic acid. Examples of the organic acid include, but are not limited to, p-toluenesulfonic acid, methanesulfonic acid, oxalic acid, p-bromobenzenesulfonic acid, carbonic acid, succinic acid, citric acid, benzoic acid, and acetic acid. Examples of the inorganic base include, but are not limited to, ammonium hydroxide, alkali metal hydroxides, alkaline earth metal hydroxides, carbonates, and hydrogencarbonates, and more specifically, sodium hydroxide, potassium hydroxide, potassium carbonate, sodium carbonate, sodium hydrogencarbonate, potassium hydrogencarbonate, calcium hydroxide, and calcium carbonate. Examples of the organic base include, but are not limited to, ethanolamine, triethylamine, and tris(hydroxymethyl)aminomethane. The method for producing these salts is not particularly limited, and for example, the compound can be produced by adding the acid or the base as described above to the compound by a known method as appropriate.

Furthermore, in the present invention, a chain substituent (e.g., a hydrocarbon group such as an alkyl group,

an unsaturated aliphatic hydrocarbon group, and the like) may be either linear or branched, unless otherwise specified, and the number of carbon thereof may be, but not limited to, for example 1 to 40, 1 to 32, 1 to 24, 1 to 18, 1 to 12, 1 to 6, or 1 to 2 (2 or more in the case of an unsaturated hydrocarbon group). In the present invention, the number of ring members (the number of atoms constituting the ring) of a cyclic group (e.g., an aryl group, a heteroaryl group, and the like) is not particularly limited, and may be, for example, 5 to 32, 5 to 24, 6 to 18, 6 to 12, or 6 to 10. When a substituent or the like has an isomer, the isomer may be any isomer unless otherwise specified, and for example, when simply referred to as a "naphthyl group", the isomer may be a 1-naphthyl group or a 2-naphthyl group.

[1. Epithelial Cancer Therapeutic Agent]

As described above, the epithelial cancer therapeutic agent of the present invention includes at least one selected from the group consisting of oxo acid, oxo acid ion, and oxo acid salt. As will be described below, the epithelial cancer therapeutic agent of the present invention may or may not include an optional component other than oxo acid, oxo acid ion, and oxo acid salt.

[1-1. Oxo Acid, Oxo Acid Ion, and Oxo Acid Salt]

In the epithelial cancer therapeutic agent of the present invention, for example, the oxo acid may be at least one selected from the group consisting of boric acid, carbonic acid, orthocarbonic acid, carboxylic acid, silicic acid, nitrous acid, nitric acid, phospholipid acid, phosphoric acid, arsenic acid, sulfurous acid, sulfuric acid, sulfonic acid, sulfinic acid, chromic acid, nichromic acid, permanganic acid, and halogenoxo acid.
The oxo acid may be, for example, a halogen oxo acid. The halogen oxo acid may be, for example, at least one selected from the group consisting of chlorine oxo acid, bromine oxo acid, and iodine oxo acid. The halogen oxo acid may be, for example, chlorine oxo acid.
The halogen oxo acid may be, for example, at least one selected from the group consisting of hypochlorous acid, chlorous acid, chloric acid, perchloric acid, hypobromous acid, bromous acid, bromic acid, perbromic acid,hypoiodous acid, iodous acid, iodic acid, andperiodic acid. The halogen oxo acid may be, for example, at least one selected from the group consisting of hypohalous acid, halous acid, halogen acid, and perhalogen acid. The halogen oxo acid may be, for example, halous acid. The halogen oxo acid may be, for example, chlorite.
In the epithelial cancer therapeutic agent of the present invention, when the oxo acid is in the form of an oxo acid salt, the oxo acid salt is not particularly limited, and may be an inorganic salt or an organic salt. The inorganic salt and the organic salt are not particularly limited, and specific examples thereof are as described above.
In the epithelial cancer therapeutic agent of the present invention, the content of at least one substance selected from the group consisting of oxo acid, oxo acid ion, and oxo acid salt is not particularly limited. For example, when the epithelial cancer therapeutic agent of the present invention does not include the radical generating catalyst to be described below, the content of the oxo acid ion relative to 1 g of the epithelial cancer therapeutic agent may be 0.1 μg or more, 1 μg or more, 2 μg or more, 3 μg or more, 5 μg or more, 10 μg or more, 100 μg or more, 200 μg or more, 500 μg or more, 1000 μg or more, 1500 μg or more, 2500 μg or more, or 5000 μg or more, or 10000 μg or less, 5000 μg or less, 2500 μg or less, 2000 μg or less, 1500 μg, 1000 μg or less, 500 μg, 100 μg or less, or 10 μg or less. When the epithelial cancer therapeutic agent of the present invention includes the radical generating catalyst to be described below, the content of the oxo acid ion relative to 1 g of the epithelial cancer therapeutic agent may be, for example, 0.1 μg or more, 1 μg or more, 2 μg or more, 3 μg or more, 5 μg or more, 10 μg or more, 100 μg or more, 200 μg, 500 μg or more, 1000 μg or more, 1500 μg or more, 2500 μg or more, or 5000 μg or more, or 10000 μg or less, 5000 μg or less, 2500 μg or less, 2000 μg or less, 1500 μg, 1000 μg or less, 500 μg, 100 μg or less, or 10 μg or less.

[1-2. Radical Generating Catalyst]

As described above, the epithelial cancer therapeutic agent of the present invention may further include a radical generating catalyst that is a substance that catalyzes radical generation from at least one substance selected from the group consisting of the oxo acid, the oxo acid ion, and the oxo acid salt, contained in the epithelial cancer therapeutic agent. Hereinafter, the radical generating catalyst may be referred to as the "radical generating catalyst of the present invention".
The radical generating catalyst of the present invention may be, for example, an organic compound or an inorganic substance. The organic substance may be, for example, at least one material selected from the group consisting of ammonium, amino acids, proteins, peptides, phospholipids, and salts thereof. The inorganic substance may include one or both of metal ions and nonmetal ions. The metal ion may include one or both of typical metal ions and transition metal ions. The inorganic substance may be, for example, at least one selected from the group consisting of alkali earth metal ions, rare earth ions, $Mg^{2+}$, $Sc^{3+}$, $Li^{+}$, $Fe^{2+}$, $Fe^{3+}$, $Al^{3+}$, silicate ions, and borate ions. Examples of the alkali earth

metal ion include ions of calcium, strontium, barium, and radium. More specifically, examples of the alkali earth metal ion include $Ca^{2+}$, $Sr^{2+}$, $Ba^{2+}$, and $Ra^{2+}$. Furthermore, the "rare earth metal" is a generic name of a set of seventeen elements, specifically, two elements such as scandium$_{21}$Sc and yttrium$_{39}$Y and fifteen elements (lanthanoids) from lanthanum$_{57}$La to lutetium$_{71}$Lu. Examples of the rare earth ion include corresponding trivalent cations of the seventeen elements.

The radical generating catalyst may be, for example, at least one selected from the group consisting of $CaCl_2$, MgClz, FeCh, $FeCl_3$, $AlCl_3$, $AlMeCl_2$, $AlMe_2Cl$, $BF_3$, $BPh_3$, $BMe_3$, $TiCl_4$, $SiF_4$, and $SiCl_4$. It is to be noted that the "Ph" represents a phenyl group, and the "Me" represents a methyl group.

The radical generating catalyst may be, for example, a Lewis acid having 0.4 eV or more. The radical generating catalyst may be, for example, Branested acid having an acid dissociation constant $pK_a$ of 5 or more as Branested acid. The upper limit of $pK_a$ is not particularly limited, and is, for example, 50 or less.

In the radical generating catalyst of the present invention, the radical generating catalyst can be selected appropriately depending on the intended use thereof, with consideration given to the reactivity, acidity, safety, and the like.

In the present invention, although the reason why the ammonium, amino acids, peptides, and phospholipids function as radical generating catalysts is not clear, it is presumably because the ammonium, amino acids, peptides, and phospholipids each have a function as a Lewis acid. In the present invention, the "Lewis acid" refers to, for example, a substance that serves as a Lewis acid with respect to the radical source.

The Lewis acidity of the radical generating catalyst of the present invention is, for example, 0.4 eV or more, 0.5 eV or more, or 0.6 eV or more. The upper limit of the Lewis acidity is not particularly limited, and is, for example, 20 eV or less. In the present invention, a criterion for determining that the Lewis acidity is equal to, greater than, or less than the above-described numerical value is, for example, whether the measured value by any one of the "Lewis acidity measuring method (1)" and the "Lewis acidity measuring method (2)" described below is equal to, greater than, or less than the above-described numerical value.

The Lewis acidity can be measured, for example, by the method described in Ohkubo, K.; Fukuzumi, S. Chem. Eur. J., 2000, 6, 4532, J. Am. Chem. Soc. 2002, 124, 10270-10271 or the method described in J. Org. Chem. 2003, 68, 4720-4726. Specifically, the Lewis acidity can be measured by the following "Lewis acidity measuring method (1)".

(Lewis acidity measuring method (1))

As to acetonitrile (MeCN) that contains cobalt tetraphenylporphyrin, saturated $O_2$, and an object whose Lewis acidity is to be measured (e.g., a cation of a metal or the like, represented by $M^{n+}$ in the following chemical reaction formula (1a)) in the following chemical reaction formula (1a), the change of the ultraviolet-visible absorption spectrum is measured at room temperature. On the basis of the obtained reaction rate constant ($k_{cat}$), the $\Delta$E value (eV), which is an indicator of the Lewis acidity, can be calculated. The higher the $k_{cat}$, the stronger the Lewis acidity. Furthermore, the Lewis acidity of an organic compound can be estimated from the energy level of the lowest unoccupied molecular orbital (LUMO) calculated by the quantum chemical calculation. The higher the value at the positive side, the stronger the Lewis acidity.

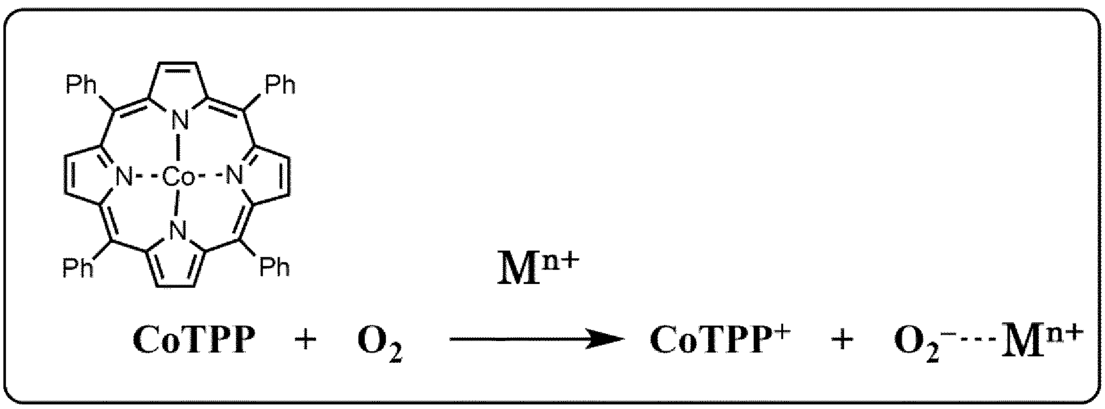

(1a)

Examples of the reaction rate constant of reaction between CoTPP and oxygen in the presence of a Lewis acid, which is an indicator of the Lewis acidity measured (calculated) by the above-described measurement method, are shown below. In the following table, the numerical value expressed in the unit "$k_{cat}$, $M^{-2}s^{-1}$" is the reaction rate constant of reaction between CoTPP and oxygen in the presence of a Lewis acid. The numerical value expressed in the unit "LUMO, eV" is the energy level of LUMO. The "benzethonium chloride" means benzethonium chloride, the "benzalkonium chloride" means benzalkonium chloride, the "tetramethylammonium hexafluorophosphate" means tetramethylammonium hexafluorophosphate, the "tetrabutylammonium hexafluorophosphate" means tetrabutylammonium hexafluorophosphate, and the "ammonium hexafluorophosphate" means ammonium hexafluorophosphate (Note from translator: in the original text in Japanese, the above sentence explains the meanings of the English terms in the table in Japanese).

[Table tpp]

| | LUMO, eV | $k_{cat}$, $M^{-2}$ $s^{-1}$ |
|---|---|---|
| benzethonium chloride | -4.12 | 0.24 |
| benzalkonium chloride | -4.02 | 0.18 |
| tetramethylammonium hexafluorophosphate | -3.58 | > 0.1 |
| tetrabutylammonium hexafluorophosphate | -2.07 | > 0.1 |
| ammonium hexafluorophosphate | -5.73 | 20 |

In the present invention, the Lewis acidity may be measured by reducing ubiquinone 1 using ubiquinone 1 (Q1), instead of oxygen molecule ($O_2$), to generate an anion radical of ubiquinone 1 in the Lewis acidity measuring method (1). In the following description, such a Lewis acidity measuring method may be referred to as the "Lewis acidity measuring method (2)". In the Lewis acidity measuring method (2), the measurement can be performed in the same manner as in the Lewis acidity measuring method (1), except that ubiquinone 1 (Q1) is used instead of oxygen molecule ($O_2$). In the Lewis acidity measuring method (2), similarly to the Lewis acidity measuring method (1), the $\Delta E$ value (eV), which is an index of Lewis acidity, can be calculated from the obtained reaction rate constant ($k_{cat}$). The Lewis acidity measuring method (2) is, for example, described in Ohkubo, K.; Fukuzumi, S. Chem. Eur. J., 2000, 6, 4532, and can be performed according to or based on the method described therein.

The Lewis acidity measuring method (2) can be performed by measuring the reaction rate constant ($k_{cat}$) with respect to the following chemical reaction formula (1b).

**Co TPP: Cobalt (II) tetraphenylporphyrin**
**Q1: Ubiquinone 1**

$$\mathrm{Co\,TPP + Q1} \xrightarrow{M^{n+}} [\mathrm{(TPP)Co}]^{+} + (\mathrm{Q1})^{\cdot -}\text{—}M^{n+} \qquad (1b)$$

in the chemical formula (1b),
$M^{n+}$ represents the radical generating catalyst,
CoTPP represents cobalt (II) tetraphenylporphyrin,
Q1 represents ubiquinone 1,
$[(TPP)Co]^{+}$ represents cobalt (III) tetraphenylporphyrin cation, and
$(Q1)^{-}$ represents an anionic radical of ubiquinone 1.

The Lewis acidity of the radical generating catalyst of the present invention may have a reaction rate constant ($k_{cat}$) for the chemical reaction formula (1b), i.e., a measured value ($K_{obs}$) of the reaction rate constant ($k_{cat}$) measured by the "Lewis acidity measuring method (2)", of, for example, $1.0 \times 10^{-5}S^{-1}$ or more, $2.0 \times 10^{-5}S^{-1}$ or more, $3.0 \times 10^{-5}S^{-1}$ or more, $4.0 \times 10^{-5}S^{-1}$ or more, $5.0 \times 10^{-5}S^{-1}$ or more, $6.0 \times 10^{-5}S^{-1}$ or more, $7.0 \times 10^{-5}S^{-1}$ or more, $8.0 \times 10^{-5}S^{-1}$ or more, $9.0 \times 10^{-5}S^{-1}$ or more, $1.0 \times 10^{-4}S^{-1}$ or more, $2.0 \times 10^{-4}S^{-1}$ or more, $3.0 \times 10^{-4}S^{-1}$ or more, $4.0 \times 10^{-4}S^{-1}$ or more, $5.0 \times 10^{-4}S^{-1}$ or more, $6.0 \times 10^{-4}S^{-1}$ or more, $7.0 \times 10^{-4}S^{-1}$ or more, $8.0 \times 10^{-4}S^{-1}$ or more, $9.0 \times 10^{-4}S^{-1}$ or more, $1.0 \times 10^{-3}S^{-1}$ or more, $2.0 \times 10^{-3}S^{-1}$ or more, $3.0 \times 10^{-3}S^{-1}$ or more, $4.0 \times 10^{-3}S^{-1}$ or more, $5.0 \times 10^{-3}S^{-1}$ or more, $6.0 \times 10^{-3}S^{-1}$ or more, $7.0 \times 10^{-3}S^{-1}$ or more, $8.0 \times 10^{-3}S^{-1}$ or more, $9.0 \times 10^{-3}S^{-1}$ or more, $1.0 \times 10^{-2}S^{-1}$ or more, $2.0 \times 10^{-2}S^{-1}$ or more, $3.0 \times 10^{-2}S^{-1}$ or more, $4.0 \times 10^{-2}S^{-1}$ or more, $5.0 \times 10^{-2}S^{-1}$ or more, $6.0 \times 10^{-2}S^{-1}$ or more, $7.0 \times 10^{-2}S^{-1}$ or more, $8.0 \times 10^{-2}S^{-1}$ or more, or $9.0 \times 10^{-2}$ $S^{-1}$or more; or $1.0 \times 10^{-1}S^{-1}$ or less, $9.0 \times 10^{-2}S^{-1}$ or less, $8.0 \times 10^{-2}S^{-1}$ or less, $7.0 \times 10^{-2}S^{-1}$ or less, $6.0 \times 10^{-2}S^{-1}$ or less, $5.0 \times 10^{-2}S^{-1}$ or less, $4.0 \times 10^{-2}S^{-1}$ or less, $3.0 \times 10^{-2}S^{-1}$ or less, $2.0 \times 10^{-2}S^{-1}$ or less, $1.0 \times 10^{-2}S^{-1}$ or less, $9.0 \times 10^{-3}S^{-1}$ or less, $8.0 \times 10^{-3}S^{-1}$ or less, $7.0 \times 10^{-3}S^{-1}$ or less, $6.0 \times 10^{-3}S^{-1}$ or less, $5.0 \times 10^{-3}S^{-1}$ or less, $4.0 \times 10^{-3}S^{-1}$ or less, $3.0 \times 10^{-3}S^{-1}$ or less, $2.0 \times 10^{-3}S^{-1}$ or less, $1.0 \times 10^{-3}S^{-1}$ or less, $9.0 \times 10^{-4}S^{-1}$ or less, $8.0 \times 10^{-4}S^{-1}$ or less, $7.0 \times 10^{-4}S^{-1}$ or less, $6.0 \times 10^{-4}S^{-1}$ or less, $5.0 \times 10^{-4}S^{-1}$ or less, $4.0 \times 10^{-4}S^{-1}$ or less, $3.0 \times 10^{-4}S^{-1}$ or less, $2.0 \times 10^{-4}S^{-1}$ or less, $1.0 \times 10^{-4}S^{-1}$ or less, $9.0 \times 10^{-5}S^{-1}$ or less, $8.0 \times 10^{-5}S^{-1}$ or less, or $7.0 \times 10^{-5}S^{-1}$ or less.

In the radical generating catalyst of the present invention, the ammonium may be, for example, quaternary

ammonium, or tertiary, secondary, primary, or zero ammonium. The ammonium is not particularly limited, and may be, for example, a nucleic acid base or the like, or an amino acid, a peptide, or the like described below.

The radical generating catalyst of the present invention may be, for example, a cationic surfactant, which may be a quaternary ammonium-type cationic surfactant. Examples of the quaternary ammonium-type cationic surfactant include benzalkonium chloride, benzethonium chloride, cetylpyridinium chloride, hexadecyltrimethylammonium bromide, dequalinium chloride, edrophonium, didecyldimethylammonium chloride, tetramethylammonium chloride, tetrabutylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, betalain, lecithin, adenine, guanine, cytosine, thymine, uracil, and cholines (e.g., choline chlorides [such as benzoyl choline chloride and a lauroylcholine chloride hydrate], phosphocholine, acetylcholine, choline, dipalmitoylphosphatidylcholine, and choline bitartrate). It is to be noted, however, that, in the radical production method of the present invention, the quaternary ammonium is not limited to a surfactant.

In the radical generating catalyst of the present invention, the ammonium may be for example, an ammonium salt represented by the following chemical formula (XI).

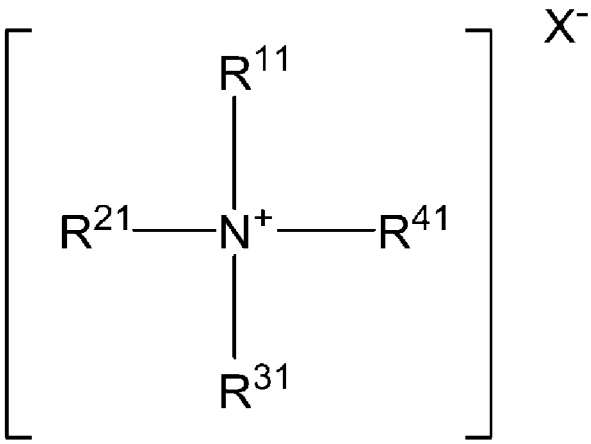

(XI)

In the chemical formula (XI), $R^{11}$, $R^{21}$, $R^{31}$, and $R^{41}$ are each a hydrogen atom or an aromatic ring, or an alkyl group, and the alkyl group may include an ether bond, a carbonyl group, an ester bond, or an amide bond, or an aromatic ring, and $R^{11}$, $R^{21}$, $R^{31}$, and $R^{41}$ may be identical to or different from each other, or two or more of $R^{11}$, $R^{21}$, $R^{31}$, and $R^{41}$ may be integrated to form a ring structure with $N^+$ to which they are bonded, and the ring structure may be saturated or unsaturated, aromatic or non-aromatic, and may or may not have one or more substituents, and $X^-$ is an anion, and $X^-$ is, for example, an anion excluding peroxodisulfate ion. In $R^{11}$, $R^{21}$, $R^{31}$, and $R^{41}$, the aromatic ring is not particularly limited, and for example, may or may not contain a heteroatom, and may or may not have a substituent. Examples of the aromatic ring containing a heteroatom (heteroaromatic ring) include a nitrogen-containing aromatic ring, a sulfur-containing aromatic ring, and an oxygen aromatic ring. Examples of the aromatic ring not containing a heteroatom include a benzene ring, a naphthalene ring, an anthracene ring, and a phenanthrene ring. Examples of the heteroaromatic ring include a pyridine ring, a thiophene ring, and a pyrene ring. The nitrogen-containing aromatic ring may or may not have a positive charge, for example. Examples of the nitrogen-containing aromatic ring having no positive charge include a pyrroline ring, a pyridine ring, a pyridazine ring, a pyrimidine ring, a pyrazine ring, a quinoline ring, an isoquinoline ring, an acridine ring, a 3,4-benzoquinoline ring, a 5,6-benzoquinoline ring, a 6,7-benzoquinoline ring, a 7,8-benzoquinoline ring, a 3,4-benzoisoquinoline ring, a 5,6-benzoisoquinoline ring, a 6,7-benzoisoquinoline ring, and a 7,8-benzoisoquinoline ring. Examples of the nitrogen-containing aromatic ring having a positive charge include a pyrrolinium ring, a pyridinium ring, a pyridazinium ring, a pyrimidinium ring, a pyrazinium ring, a quinolinium ring, an isoquinolinium ring, an acridinium ring, a 3,4-benzoquinolinium ring, a 5,6-benzoquinolinium ring, a 6,7-benzoquinolinium ring, a 7,8-benzoquinolinium ring, a 3,4-benzoisoquinolinium ring, a 5,6-benzoisoquinolinium ring, a 6,7-benzoisoquinolinium ring, and a 7,8-benzoisoquinolinium ring. The oxygen-containing aromatic ring or the sulfur-containing aromatic ring may be, for example, an aromatic ring in which at least one of a carbon atom or a nitrogen atom of the above-described heteroatom-free aromatic ring or nitrogen-containing aromatic ring is substituted with at least one of an oxygen atom or a sulfur atom. In $R^{11}$, $R^{21}$, $R^{31}$, and $R^{41}$, when the alkyl group or the aromatic ring has a substituent, the substituent is not particularly

limited and is optional, and examples thereof include a sulfo group, a nitro group, and a diazo group.

The ammonium salt represented by the chemical formula (XI) may be an ammonium salt represented by the following chemical formula (XII), for example.

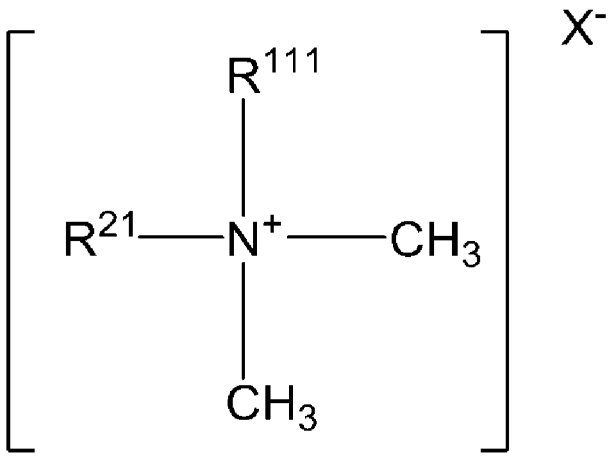

(XII)

In the chemical formula (XII), $R^{111}$ is an alkyl group having 5 to 40 carbon atoms and may include an ether bond, a ketone (carbonyl group), an ester bond, or an amide bond, substituent, or an aromatic ring, and $R^{21}$ and $X^-$ are the same as those in the chemical formula (XI). In $R^{111}$, the aromatic ring is not particularly limited, and may or may not contain a heteroatom, and may or may not have a substituent, for example. In $R^{111}$, the aromatic ring is not particularly limited, and specific examples are the same as those in $R^{11}$, $R^{21}$, $R^{31}$, and $R^{41}$ of the chemical formula (XI). In $R^{111}$, when the alkyl group or the aromatic ring has a substituent, the substituent is not particularly limited, is optional, and is, for example, the same as those in $R^{11}$, $R^{21}$, $R^{31}$, and $R^{41}$ of the chemical formula (XI).

In the chemical formula (XII), $R^{21}$ may be, for example, a methyl group or a benzyl group. In the benzyl group, one or more hydrogen atoms of the benzene ring may or may not be substituted with any substituent. The substituent may be, for example, an alkyl group, an unsaturated aliphatic hydrocarbon group, an aryl group, a heteroaryl group, a halogen, a hydroxy group (-OH), a mercapto group (-SH), or an alkylthio group (-SR, where R is an alkyl group).

The ammonium salt represented by the chemical formula (XII) may be, for example, an ammonium salt represented by the following chemical formula (XIII).

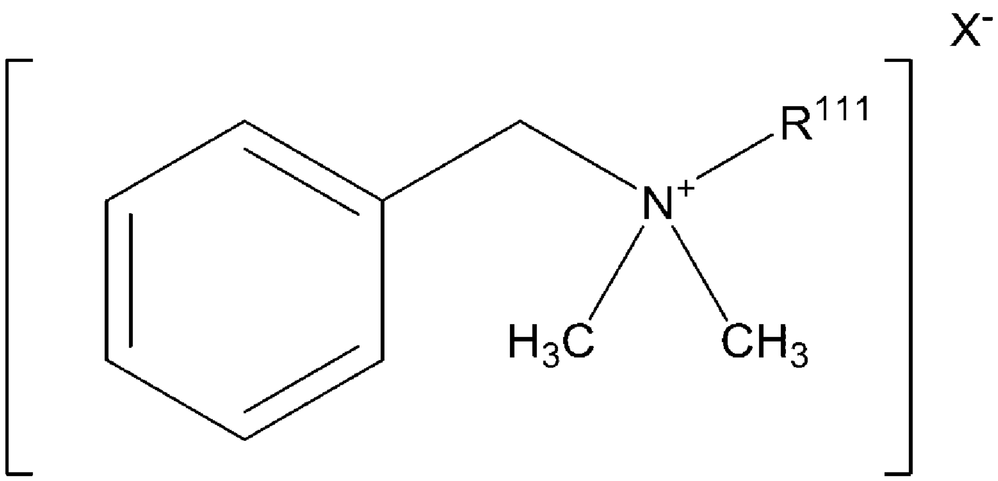

(XIII)

In the chemical formula (XIII), $R^{111}$ and $X^-$ are the same as those in the chemical formula (XII).

The ammonium may be, for example, at least one selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, betalain, lecithin, adenine, guanine, cytosine, thymine, uracil, and cholines. The ammonium may be, for example, benzethonium chloride.

The ammonium salt represented by the chemical formula (XI) may be, for example, an ammonium salt represented by the following chemical formula (XIV).

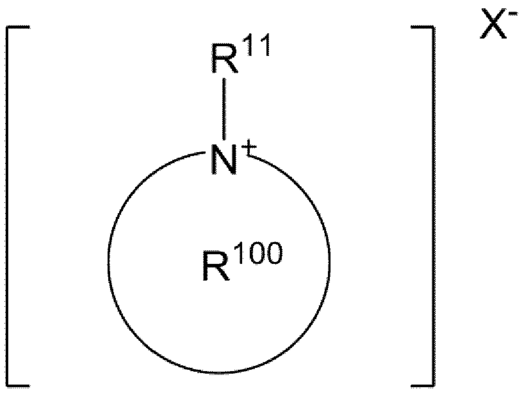

(XIV)

Where in the chemical formula (XIV), $R^{100}$ may form a ring structure, which may be saturated or unsaturated, aromatic or non-aromatic, and may or may not have one or more substituents, and $R^{11}$ and $X^-$ are the same as those in the chemical formula (XI).

The ammonium salt represented by the chemical formula (XI) may be, for example, an ammonium salt represented by the following chemical formula (XV).

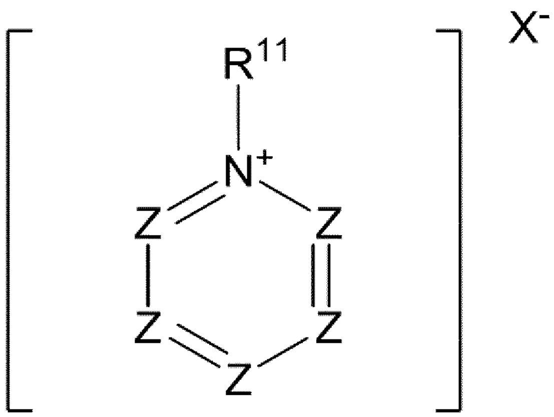

(XV)

In the chemical formula (XV), Zs are each CH or N, may be identical to or different from each other, and in the case of CH, H may be substituted with a substituent, and $R^{11}$ and $X^-$ are the same as those in the chemical formula (XI).

The ammonium salt represented by the chemical formula (XI) may be, for example, an ammonium salt represented by the following chemical formula (XVI).

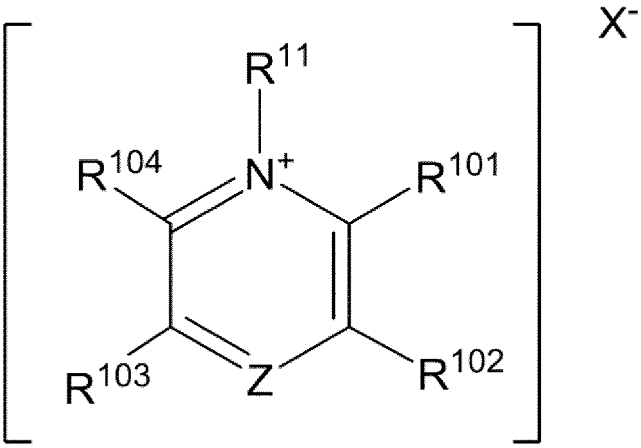

(XVI)

In the chemical formula (XVI), $R^{101}$, $R^{102}$, $R^{103}$, and $R^{104}$ are each a hydrogen atom or a substituent, and $R^{101}$, $R^{102}$, $R^{103}$, and $R^{104}$ may be identical to or different from each other, or two or more of $R^{101}$, $R^{102}$, $R^{103}$, and $R^{104}$ may be integrated to form a ring structure with $N^+$ to which they are bonded, and the ring structure may be saturated or unsaturated, and aromatic or non-aromatic, and may or may not have one or more substituents, Z is CH or N, and in the case of CH, H may be substituted with a substituent, and $R^{11}$ and $X^-$ are the same as those in the chemical formula (XI).

The ammonium salt represented by the chemical formula (XI) may be, for example, an ammonium salt represented by the following chemical formula (XVII).

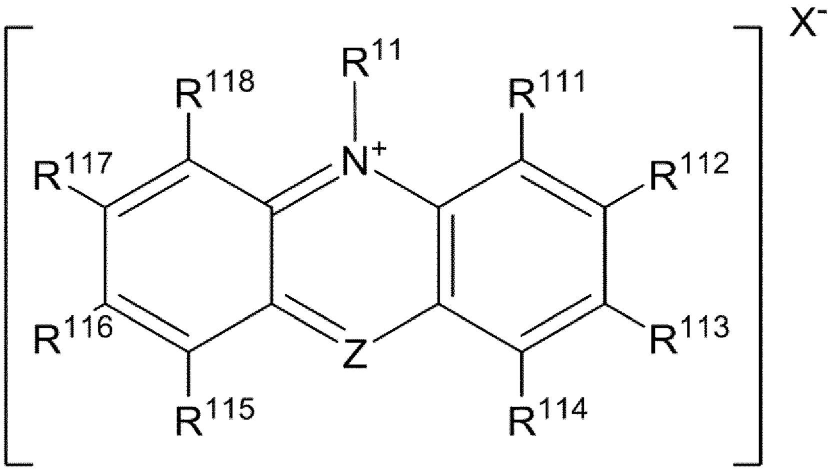

(XVII)

In the chemical formula (XVII), $R^{111}$ to $R^{118}$ are each a hydrogen atom or a substituent, and may be identical to or different from each other, or two or more of $R^{111}$ to $R^{118}$ may be integrated to form a ring structure, which may be aromatic or non-aromatic and may or may not have one or more substituents, Z is CH or N, and in the case of CH, H may be substituted with a substituent, and $R^{11}$ and $X^-$ are the same as those in the chemical formula (XI).

The ammonium salt represented by the chemical formula (XI) may be, for example, at least one selected from the group consisting of benzethonium chloride, benzalkonium chloride, hexadecyltrimethylammonium chloride, tetramethylammonium chloride, ammonium chloride, methylammonium chloride, and tetrabutylammonium chloride. It is particularly preferable that the ammonium salt represented by the chemical formula (XII) is benzethonium chloride.

Benzethonium chloride ($Bzn^+Cl^-$) can be, for example, represented by the following chemical formula. Benzalkonium chloride can be, for example, a compound represented by the chemical formula (XIII) where $R^{111}$ is an alkyl group having 8 to 18 carbon atoms and $X^-$ is a chloride ion.

Bzn+Cl-

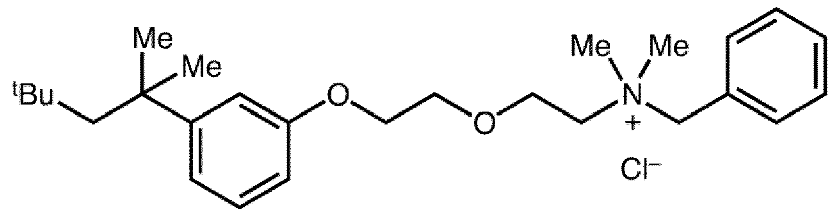

In the chemical formulae (XI), (XII), (XIII), (XIV), (XV), (XVI), and (XVII), $X^-$ may be any anion and is not particularly limited. $X^-$ is not limited to a monovalent anion, and may be an anion with any valence, such as a divalent anion or a trivalent anion. When the anion is an anion with a plurality of electric charges, such as a divalent anion or a trivalent anion, the number of molecules of the (monovalent) ammonium in each of the chemical formulae (XI), (XII), (XIII), (XIV), (XV), (XVI), and (XVII) is determined by, for example, the number of molecules of the anion × the valence of the anion (e.g., when the anion is divalent, the number of molecules of the (monovalent) ammonium is twice the number of molecules of the anion). $X^-$ may be, for example, a halogen ion (a fluoride ion, a chloride ion, a bromide ion, or an iodide ion), an acetate ion, a nitrate ion, or a sulfate ion.

In the present invention, the radical generating catalyst is not limited to the chemical formulae (XI), (XII), (XIII), (XIV), (XV), (XVI) and (XVII), and may be ammonium having any structure containing an aromatic ring. The aromatic ring is not particularly limited, and may be, for example, an aromatic ring exemplified in $R^{11}$, $R^{21}$, $R^{31}$, and $R^{41}$ of the chemical formula (XI).

In the present invention, the radical generating catalyst may be, for example, a sulfonic acid amine or ammonium thereof. The sulfonic acid amine is, for example, amine having a sulfonic group (sulfonic acid group) in its molecule. Examples of the sulfonic acid amine include taurine, sulfamic acid, 3-amino-4-hydroxy-1-naphthalenesulfonic acid, sulfamic acid, p-toluidine-2-sulfonic acid, o-anisidine-5-sulfonic acid, direct blue 14, 3- [N, N-bis (2-hydroxyethyl)amino]-2-hydroxypropanesulfonic acid, 3-[(3-colamidopropyl)dimethylammonio]-1-propanesulfonate, aminomethanesulfonic acid, 3-sulfopropylamine, 2-aminobenzenesulfonic acid, R(+)-3-aminotetrahydrofuran, toluene, 4-amino-5-hydroxy-1,7-naphthalenedisulfonic acid, N-(2-acetamido)-2-aminoethanesulfonic acid, 4'-amino-3'-methoxyazobenzene-3-sodium sulfonate, Lapatinib ditosylate, N-tris (hydroxymethyl) methyl-2-aminoethanesulfonic acid, 8-amino-1,3,6-naphthalenetrisulfonic acid disodium hydrate, 1-aminonaphthalene-2-sulfonic acid, (2S,3S)-3-Amino-2-methyl-4-oxo-1-azetidinesulfonic acid, sodium 3- (1-naphthylamino) propanesulfonate, 3-methyl-4-aminobenzenesulfonic acid, sodium 3- Cyclohexylamino-2-hydroxypropanesulfonic acid, sodium N-tris (hydroxymethyl) methyl-2-aminoethanesulfonic acid, 4-amino-1-naphthalenesulfonic acid, sodium sulfamate, tricaine, sodium sulfanilate, 1,4-phenylenediamine 2-sulfonic acid, p- anisidine-2-sulfonic acid, 6-amino-1-naphthalenesulfonic acid, 3,4-diaminobenzene sulfonic acid, 3-amino-4-chlorobenzene sulfonic acid, 3-[(4-Amino-3-methylphenyl) azo] benzenesulfonic acid, 3-amino-4-hydroxy-5-nitrobenzenesulfonic acid, 5-amino-6-hydroxy-3-nitrobenzenesulfonic acid, 4-acetamide-2-Aminobenzenesulfonic acid hydrate, 2-aminophenol-4-sulfonic acid, 1-amino-2-methoxy-5-methyl-4-benzenesulfonic acid, dansylic acid, Sulfamic acid [(1S, 2S, 4R)-4-[4-[[(1S)-2,3-dihydro-1H-inden-1-yl] amino]-7H-pyrrolo [2,3-d] pyrimidin-7-yl]-2- hydroxycyclopropyl] methyl ester, 5-sulfo-4'-diethylamino-2,2' dihydroxyazobenzene, 2-aminonaphthalene-6,8-disulfonic acid, sodium 2- [N,N-bis (2-hydroxyethyl) amino]-1-ethanesulfonate, 3-acetyl-2- (methylaminosulfonyl) thiophene, sodium 4-amino-2-chlorotoluene-5-sulfonate, 5- (3-amino-5-oxo-2-pyrazolin-1-yl)-2-phenoxybenzenesulfonic acid, potassium sulfamate, p-aminoazobenzene monosulfonic acid, 3-[(3-Cholamidopropyl) dimethylamino]-2-hydroxy-1-propanesulfonate, 3-amino-2,7-naphthalenes disulfonic acid monosodium, 3- [N, N-bis (hydroxyethyl) amino]-2-hydroxypropanesulfonic acid sodium salt, di (amidosulfuric acid) cobalt (II), 3- (4-amino-3-methoxyphenylazo) benzenesulfonic acid, Nickel (II) sulfamate tetrahydrate, sodium 2,4-diaminobenzenesulfonate, 5-amino-2-chlorotoluene-4-sulfonic acid, 2,5-dichlorosulfanilic acid, 4-methylbenzenesulfonic acid, APTS (aminopyrenetrisulfonic acid), 4'-aminoazobenzene-3-sulfonic acid, Pontacyl carmine 2B, p-anisidine-3-sulfonic acid, 4,4'-bis (4-amino-1-naphthylazo)- 2,2'-stilbenesulfonic acid, 3-aminonaphthalene-8-hydroxy-4, 6-disulfonic acid, sodium 4-amino-1,5-naphthalenedisulfonate, sodium 4-aminoazobenzene-4'-sulfonate, 5-amino-2-methylbenzenesulfonic acid, disodium 7-amino-1,3-naphthalene disulfonate, alizarin safilol SE, sodium 7-amino-2-naphthalenesulfonate, 6-amino-5-bromopyridine-3-sulfonic acid, 2-aminoethanethiol p-toluenesulfonate, sodium 2-amino 1-naphthalenesulfonate, 6-amino-1,3-naphthalenedisulfonic acid disodium hydrate, N,N,N',N'-tetraethylsulfamide, 5-amino-2-ethoxybenzenesulfonic acid, 3,5-diamino-2,4,6-trimethylbenzenes phosphonic acid, 7-amino-1-naphthalenesulfonic acid, sulfamic acid, guanidine, 2-amino-5-nitrobenzenesulfonic acid, nickel (II) diamide sulfate, 4-amino-4'-nitrostilbene-2,2'-disulfonic acid disodium, aniline-2,5-disulfonic acid monosodium, 5-amino-1-naphthol-3-sulfonic acid hydrate, 2,5-dichlorosulfanilic acid sodium salt, 6-aminohexanoic acid hexyl p-toluenesulfonate, rac- (R*)-2- (4-chlorophenyl)-3-amino-1-propanesulfonic acid, 2- (N,N-dipropyl) amino anisole-4-Sulfonic acid, 2-amino-4-chlorophenol-6-sulfonic acid, 6-amino-1,3-naphthalenedisulfonic acid, 5,10,15,20-tetrakis [4-(trimethylammonio) phenyl]- 21H,-23H-porphine tetratosylate, 5-amino-2-[(4-aminophenyl) amino] benzenesulfonic acid, 4-amino-3-chlorobenzenesulfonic acid, 2-aminobenzenesulfonic acid phenyl ester, 4- Acetylamino-4'-isothiocyanatostilbene-2,2'-disulfonic acid disodium salt, (S)-3-amino-2-oxetanone p-toluenesulfonic acid salt, 5-acetylamino-4-hydroxy-2,7-naphthalenedisulfonic acid disodium salt, 2-phenylamino-5-aminobenzenesulfonic acid, sodium 4-octadecylamino-4- oxo-2-[(sodiooxy)sulfonyl]butanoate, and 3,5-diamino-4-methylbenzenesulfonic acid.

In the present invention, the radical generating catalyst may be, for example, nicotinic amine or ammonium thereof. The nicotinic amine is, for example, amine having a ring structure in a molecule, and the ring structure has a nicotine skeleton. Examples of the nicotinic amine include nicotinamide and alkaloid.

In the present invention, the radical generating catalyst may be, for example, nitrite amine or nitrite ammonium. The nitrite amine or nitrite ammonium is, for example, a compound obtained by reacting amine with nitrous acid or a nitrous acid derivative. Examples of the nitrite amine or nitrite ammonium include diazo compounds, diazonium salts, N-nitroso compounds, and C-nitroso compounds.

In the radical generating catalyst of the present invention, the ammonium may include a plurality of ammonium structures ($N^+$) in one molecule. Further, the ammonium may, for example, form a dimer, trimer, or the like by association of a plurality of molecules through a $\pi$ electron interaction.

In the radical generating catalyst of the present invention, the amino acid is not particularly limited. The amino acid may contain, for example, at least one of both an amino group or an imino group, or a carboxy group in the molecule. The amino acid may be, for example, an $\alpha$-amino acid, a $\beta$-amino acid, a $\gamma$-amino acid, or any other amino acid. The amino acid may be, for example, an amino acid constituting protein, and specifically may be at least one selected from the group consisting of, for example, glycine, alanine, valine, leucine, isoleucine, serine, threonine, aspartic acid, glutamic acid, asparagine, glutamine, lysine, hydroxylysine, arginine, cysteine, cystine, methionine, phenylalanine, tyrosine, tryptophan, histidine, proline, and 4-hydroxyproline.

In the radical generating catalyst of the present invention, the peptide is not particularly limited. The peptide may be, for example, one in which two or more of the amino acid molecules are linked by a peptide bond. The peptide may be, for example, at least one of oxidized glutathione (GSSG) or reduced glutathione (GSH).

In the radical generating catalyst of the present invention, the phospholipid is not particularly limited. The phospholipid may be, for example, a lipid containing phosphorus atoms in the molecule, and may be, for example, a lipid containing a phosphate ester bond (P-O-C) in the molecule. The phospholipid may or may not have, for example, at least one of an amino group, an imino group, an ammonium group, or an iminium group, in the molecule. The phospholipid may be, for example, at least one selected from the group consisting of phosphatidylserine, phosphatidylcholine, phosphatidic acid, phosphatidylethanolamine, phosphatidylglycerol, and cardiolipin.

As described above, the radical generating catalyst of the present invention is a substance that catalyzes radical generation from at least one substance selected from the group consisting of the oxo acid, the oxo acid ion, and the oxo acid salt, contained in the epithelial cancer therapeutic agent. Therefore, while the radical generating catalyst of the present invention may catalyze radical generation from a radical source *in vitro,* for example, it is preferable to catalyze radical generation from at least one substance selected from the group consisting of the oxo acid, oxo acid ion, and oxo acid salt, *in vivo.* The living body may be, for example, a human body, or may be a body of an animal other than a human.

The radical generating catalyst of the present invention may, for example, catalyze the radical generation from a radical source in a digestive organ. The digestive organ may be, for example, at least one selected from the group consisting of an oral cavity, a pharynx, an esophagus, a stomach, a duodenum, a small intestine, and a large intestine. The digestive organ may be, for example, the large intestine. The small intestine may be, for example, at least one selected from the group consisting of duodenum, jejunum and ileum. The large intestine may be at least one selected from the group consisting of, for example, cecum, colon and rectum. The radical generating catalyst of the present invention may be used, for example, for sterilizing in the digestive organ, induction of changes in intestinal bacterial flora, treatment or suppression of symptoms of ulcerative colitis, and the like.

In the epithelial cancer therapeutic agent of the present invention, the content of the radical generating catalyst is not particularly limited. The content of the radical generating catalyst relative to 1 g of the epithelial cancer therapeutic agent may be, for example, 0.1 $\mu$g or more, 1 $\mu$g or more, 2 $\mu$g or more, 3 $\mu$g or more, 5 $\mu$g or more, 10 $\mu$g or more, 100 $\mu$g or more, 200 $\mu$g, 500 $\mu$g or more, 1500 $\mu$g or more, 2500 $\mu$g or more, or 5000 $\mu$g or more, or 10000 $\mu$g or less, 5000 $\mu$g or less, 2500 $\mu$g or less, 2000 $\mu$g or less, 1000 $\mu$g or less, 500 $\mu$g or 100 $\mu$g or less, or 10 $\mu$g or less per.

[1-3. Other Optional Component]

As described above, the epithelial cancer therapeutic agent of the present invention includes at least one selected from the group consisting of oxo acid, oxo acid ion, and oxo acid salt. The epithelial cancer therapeutic agent of the present invention may or may not include an optional component other than the oxo acid, oxo acid ion, and oxo acid salt. The optional component may be, for example, the radical generating catalyst. The epithelial cancer therapeutic agent of the present invention may or may not include an optional component other than the radical generating catalyst. The optional component other than the radical generating catalyst is not particularly limited. Specific examples thereof are described in "2. Form, Production Method, Use Method, etc. of Epithelial Cancer Therapeutic Agent" below.

[2. Form, Production Method, Use Method, etc. of Epithelial Cancer Therapeutic Agent]

The form of the epithelial cancer therapeutic agent of the present invention is not particularly limited, and may be, for example, solid, powder, semi-solid, liquid, or the like. In the case of solid, the form may be, for example, a tablet, a capsule, or the like.

The method for producing the epithelial cancer therapeutic agent of the present invention is not particularly limited, and the epithelial cancer therapeutic agent can be produced, for example, by a method the same as or according to a general method for producing a drug. Specifically, for example, all the components of the epithelial cancer therapeutic agent of the present invention may be simply mixed. Further, for example, all the components after being mixed may be, for example, compacted into a tablet, or encapsulated in a capsule.

As described above, the epithelial cancer therapeutic agent of the present invention may or may not include an optional component other than the radical generating catalyst. As described above, the optional component is not particularly limited, and may be, for example, the same as those in drugs generally or similar to those. When the epithelial cancer therapeutic agent of the present invention is in a liquid state, examples of the optional component include water, a pH buffer solution, and physiological saline.

The epithelial cancer therapeutic agent of the present invention may further include, for example, one or more pharmaceutically acceptable additives as the optional component. That is, for example, the sulfated polysaccharide or sulfated oligosaccharide may be formulated together with one or more pharmaceutically acceptable additives prior to administration. Examples of the additive include, but are not limited to, inert substances such as carriers, diluents, flavors, sweeteners, lubricants, dissolving agents, suspending agents, binders, tablet disintegrating agents, and encapsulating materials. In addition to these, for example, any additives commonly used in the field of pharmaceuticals may be used as appropriate.

When the epithelial cancer therapeutic agent of the present invention is orally administered, for example, the following substances can be used as the additives. Examples of the carrier include lactose, starch, sucrose, glucose, sodium carbonate, mannitol, sorbitol, calcium carbonate, calcium phosphate, calcium sulfate, and methylcellulose. Examples of the disintegrating agent include corn flour, starch, methylcellulose, agar, bentonite, xanthan gum, and alginic acid. Examples of the binder include gelatin, natural sugar, beta-lactose, corn sweetener, natural and synthetic gums, acacia, tragacanth, sodium alginate, carboxymethylcellulose, polyethylene glycol, and wax. Examples of the lubricant include magnesium stearate, sodium stearate, stearic acid, sodium oleate, sodium benzoate, sodium acetate, salt, and talc.

As to the epithelial cancer therapeutic agent of the present invention, at least one selected from the group consisting of oxo acid, oxo acid ion, and oxo acid salt, and the radical generating catalyst and other optional components, if necessary, may be mixed with a diluent or encapsulated in a carrier. The carrier can be in the form of, for example, a capsule, sachet, paper, or other container. The carrier may also serve as a diluent, and may be solid, semi-solid, or a liquid that acts as a vehicle. The form of the pharmaceutical of the present invention is not particularly limited, and may be various forms such a tablet, a pill, powder, a lozenge, an elixir, a suspension, an emulsion, a solution, a syrup, an aerosol, an ointment, a soft or hard gelatin capsule, a suppository, a sterilize injectable solution, and sterile packaged powder.

The dosage form of the epithelial cancer therapeutic agent of the present invention is not particularly limited, and the epithelial cancer therapeutic agent may be administered orally or parenterally, depending on the intended use thereof. The form of the epithelial cancer therapeutic agent when administered as an oral preparation is not particularly limited, and ordinary forms such as an enteric-coated tablet, a capsule, a pill, powder, a granule, an elixir, a tincture, a solution, a suspension, a syrup, a solid or liquid aerosol, and an emulsion, and the forms commonly used by those skilled in the art can be selected. In addition, the form of the epithelial cancer therapeutic agent when administered parenterally. is not particularly limited, and the forms commonly used by those skilled in the art, such as intravenous administration, intraperitoneal administration, subcutaneous administration, intramuscular administration, injection, infusion, and the like, can be selected.

In addition, the dose and the administration interval of the epithelial cancer therapeutic agent of the present invention are not particularly limited, and may be appropriately selected depending on the intended use thereof. They can be selected by those skilled in the art with consideration given to a variety of factors including the age, weight, sex, medical condition, disease state, route of administration, level of metabolic and excretory function of the patient, dosage form used, particular oxo acid, oxo acid ion or oxo acid salt administered, and the like. The dose concentration of the epithelial cancer therapeutic agent of the present invention per administration in the case of topical administration may be, for example, 100 ppm (0.1% by mass) or more, 200 ppm or more, 300 ppm or more, 500 ppm or more, 1000 ppm or more, 2500 ppm or more, or 5000 ppm or more, for example, 5000 ppm (3% by mass) or less, 2500 ppm or less, 1000 ppm or less, 500 ppm or less, 300 ppm or less, or 200 ppm or less. In the case of topical administration, the administration interval of the epithelial cancer therapeutic agent of the present invention may be, for example, 1 day or more, 2 days or more, 3 days or more, 4 days or more, 5 days or more, 6 days or more, or 7 days or more, and may be,

for example, 7 days or less, 6 days or less, 5 days or less, 4 days or less, 3 days or less, or 2 days or less. For example, for bladder cancer, in order to prevent recurrence, the epithelial cancer therapeutic agent of the present invention may be administered immediately after transurethral resection and administered once a week for 4 to 8 weeks (administration volume: 10 to 40 ml, for example, 30 ml).

The oxo acid, oxo acid ion, or oxo acid salt, which is an active ingredient of the epithelial cancer therapeutic agent of the present invention, is less toxic than a general epithelial cancer therapeutic agent. In addition, since the toxicity of oxo acid, oxo acid ion, or oxo acid salt, which is an active ingredient of the epithelial cancer therapeutic agent of the present invention, is well known, it is possible to select a safe dosage and administration interval for the patient. Therefore, according to the present invention, for example, it is possible to provide an epithelial cancer therapeutic agent having a very small side effect compared with a commonly used anticancer agent or a BCG preparation. Furthermore, according to the epithelial cancer therapeutic agent of the present invention, it is also possible to obtain, for example, a strong recurrence inhibitory effect (recurrence preventing effect). That is, according to the epithelial cancer therapeutic agent of the present invention, for example, the recurrence rate of epithelial cancer can be extremely reduced.

The epithelial cancer therapeutic agent of the present invention can also be used, for example, as an epithelial cancer cell apoptosis promoting agent. For example, by promoting apoptosis of epithelial cancer cells, proliferation of the epithelial cancer cells can be inhibited more effectively.

Further, the present invention also provides a method for producing the epithelial cancer therapeutic agent of the present invention using at least one selected from the group consisting of oxo acid, oxo acid ion, and oxo acid salt. The epithelial cancer therapeutic agent production method of the present invention is specifically as described above. In the epithelial cancer therapeutic agent production method of the present invention, as described above, the radical generating catalyst may or may not be used, or an optional component other than the radical generating catalyst may or may not be used. Further, the present invention also provides a method for treating epithelial cancer patient, including administering to the patient the epithelial cancer therapeutic agent of the present invention. In the administering, the administration method, the dosage, the administration interval, and the like are not particularly limited, and are, for example, those as described above.

The subject to be administered the epithelial cancer therapeutic agent of the present invention or the patient to be treated according to the present invention is, for example, a human or a non-human animal excluding a human. Examples of the non-human animal include mice, rats, rabbits, monkeys, dogs, and cows.

Examples

Hereinafter, examples of the present invention will be described. The present invention, however, is not limited to the following examples.

[Example 1: Bladder Cancer Therapeutic Agent using MA-T]

The bladder cancer therapeutic agent (the epithelial cancer therapeutic agent of the present invention) was produced as follows, and the effect thereof was verified. In the following description, an aqueous solution (drug) containing $NaClO_2$ and benzethonium chloride may be referred to as "MA-T". In MA-T, the concentrations of $NaClO_2$ and benzethonium chloride are not particularly limited, and the same amounts of $NaClO_2$ and benzethonium chloride can be used, for example. In the following description, for example, an aqueous solution (drug) containing 100 ppm $NaClO_2$ and 100 ppm benzethonium chloride may be referred to as "100 ppm MA-T". Since MA-T contains $NaClO_2$, it functions as the epithelial cancer therapeutic agent of the present invention. In addition, in the following description, the epithelial cancer therapeutic agent of the present invention produced using MA-T may be referred to as "MA-T epithelial cancer therapeutic agent of the present invention".

[(1) Cell Culture]

Bladder cancer cell line UM-UC-3 was obtained from American Type Culture Collection (ATCC), bladder cancer cell line BOY-12E was obtained from Tohoku University, and bladder cancer cell lines UM-UC-2, 5637, and EJ-1 were obtained from Nara Medical University.

UM-UC-3, UM-UC-2, and EJ-1 cells were cultured at 37°C under 5% $CO_2$ using a medium, which was obtained by adding Fetal bovine serum (FBS, Biosera) to D-MEM (Low glucose, trade name, FUJIFILM Wako Pure Chemical Corporation) so as to become 10% by volume, 5637 cells were cultured at 37°C under 5% $CO_2$ using a medium, which was obtained by adding Fetal bovine serum (FBS, Biosera) to RPMI-1640 (trade name, FUJIFII,M Wako Pure Chemical Corporation) so as to become 10% by volume, and BOY-12E cells were cultured at 37°C under 5% $CO_2$ using a medium, which was obtained by adding Fetal bovine serum (FBS, Biosera) to E-MEM (trade name, FUJIFII,M Wako Pure Chemical Corporation) so as to become 10% by volume. When the cells were passaged, 0.025% Trypsin/EDTA (ethylenediaminetetraacetic acid) was added to the cells and incubated at 37°C under 5% $CO_2$ to detach the cells, and then centrifuged at 1500 rpm for 3 minutes to recover the cells.

[(2) Reagent Preparation]

1 × PBS aqueous solution of $NaClO_2$ and 1 × PBS aqueous solution of benzethonium chloride were prepared by diluting a 10000 ppm $NaClO_2$ (sodium chlorite) aqueous solution with 10 × phosphate buffered saline (PBS) and distilled water (DW) and by diluting a 10000 ppm benzethonium chloride aqueous solution with 10 × phosphate buffered saline (PBS) and distilled water (DW) so as to eventually became 1 × PBS as shown in the table below. These aqueous solutions correspond to the epithelial cancer therapeutic agent of the present invention containing MA-T, i.e., the "MA-T epithelial cancer therapeutic agent of the present invention". In the PBS, the "10×" represents a 10-fold concentration, and the "1×" represents a 1-fold concentration. Table 1 below shows the amount of each solution required when the total amount of the prepared aqueous solution is 400 μL.

[Table 1]

| Concentration [ppm] | 10000 ppm $NaClO_2$ aqueous solution [μL] | 10000 ppm benzethonium chloride aqueous solution [μL] | 10 × PBS [μL] | DW [μL] |
|---|---|---|---|---|
| 0 | 0 | 0 | 40 | 360 |
| 12.5 | 0.5 | 0.5 | 40 | 359 |
| 25 | 1 | 1 | 40 | 358 |
| 50 | 2 | 2 | 40 | 356 |
| 100 | 4 | 4 | 40 | 352 |

FIG. 1 shows graphs showing the results of concentration-and reaction time-dependent analysis of the effect of a mixed aqueous solution of $NaClO_2$ and benzethonium chloride on the proliferation of UM-UC-3 and 5637 cells. The horizontal axis indicates time (hr) and the vertical axis indicates the number of cells in normalized indices. The analysis results by xCelligence were normalized with the values immediately after the additions and expressed as "normalized cell index". As shown in FIG. 1, even when the concentration of the mixed aqueous solution of $NaClO_2$ and benzethonium chloride was low such as 25 ppm, the proliferation of bladder cancer cells was inhibited. Further, when the concentrations of the mixed aqueous solution of $NaClO_2$ and benzethonium chloride were high, the proliferation of bladder cancer cells was further inhibited, and when the concentrations of the mixed aqueous solution of $NaClO_2$ and benzethonium chloride were at 100 ppm, the proliferation of all the cells was completely inhibited.

[(3) Time-Lapse Photography]

UM-UC-3 cells were seeded in a 3 cm dish at 1 × $10^4$ cells/1000 ml/dish, the medium was removed on the next day, and then 1000 μL of a 100 ppm MA-T aqueous solution was added per well and incubated for 120 minutes. After the MA-T aqueous solution was removed, normal culture medium was added to all the wells, and the same field of view was photographed every 10 minutes for 48 hours with a 20× lens using a BZ-X710 (trade name, KEYENCE CORPORATION.) equipped with a microscope incubation system (trade name, Tokai Hit., Co, Ltd.). The obtained images were processed into a movie using the software built in the system. As a Control, the time-lapse photography was performed in the same manner using a sample treated in the same manner, except that PBS was used instead of the MA-T aqueous solution.

The photographs of FIGs. 2 and 3 show the results of the time-lapse photography. FIG. 2 shows photographs taken immediately after the start of photographing (0-hour culture), and FIG. 3 shows photographs taken 48 hours after the start of photographing (48-hour culture). In each of FIGs. 2 and 3, the "Control" represents a PBS-added group (Control), and the "MA-T" represents a 100 ppm MA-T sample (i.e., a sample to which the MA-T epithelial cancer therapeutic agent of the present invention was added). As shown in FIGs. 2 and 3, in the PBS-added group (Control), the proliferation of UM-UC-3 cells was observed 48 hours after the start of photographing, whereas the proliferation of UM-UC-3 cells to which the MA-T epithelial cancer therapeutic agent of the present invention was added was inhibited. That is, it was verified that the MA-T epithelial cancer therapeutic agent of the present invention is effective in inhibiting the proliferation of bladder cancer cells.

[(4) Apoptosis Analysis]

The above cells were seeded in a six-well plate. UM-UC-3 cells were seeded at 5 × $10^4$ cells/well, and 5637 cells were seeded at 8× $10^4$ cells/well. The culture medium was removed on the next day, and 100 ppm MA-T aqueous solution was added. Thereafter, the resultant was incubated at 37°C under 5%$CO_2$ (12 hours for UM-UC-3 cells and 6 hours for 5637 cells), and then the cells including the supernatant were recovered and centrifuged at 3000 rpm for 5 minutes to obtain cell pellets. After the pellet was washed twice with 1 × PBS, 294 μL of 1 × Binding buffer, 3 μL of Propidium iodide, and 3 μL of Annexin V of Annexin V-FITC Apoptosis Detection Kit (trade name, Bio Vision) were added, and the pellets were suspended. The suspension was passed through a 37 μm nylon mesh (NBC MeshTech Inc.) and transferred to a round bottom tube (Falcon). Thereafter, the population of each of the stained cells was analyzed by FACS Calibur (trade name, Becton, Dickinson and Company). The single stained sample of Propidium iodide and the single stained sample of Annexin V were prepared for FACS voltage control. As a Control, the apoptotic analysis was performed in the same manner using samples treated in the same manner except that PBS was used.

FIG. 4 shows apoptotic analysis of 5637 cells. In FIG. 4, the "Control" represents a PBS sample (Control), and the "MA-T" represents a 100 ppm MA-T sample (i.e., a sample to which the MA-T epithelial cancer therapeutic agent of the present invention was added). As shown in FIG. 4, the apoptotic rate of 5637 cells was 8.5% in the PBS sample (Control), whereas the apoptotic rate of 5637 cells was 30.2% in the MA-T sample to which the MA-T epithelial cancer therapeutic agent of the present invention was added. That is, it was verified that the epithelial cancer therapeutic agent of the present invention had an effect of promoting apoptosis of epithelial cancer cells.

[(5) Antitumor Effect of MA-T using Luciferase-Expressing Bladder Cancer Cell Orthotopic Transplantation Model Mouse]

The evaluation was performed using an orthotopic transplantion model mouse with luciferase-expressing bladder cancer cells, a proprietary technology. Specifically, luciferase-expressing human bladder cancer UM-UC-3 cells were produced, a luciferase substrate was administered to a mouse 10 days after transplantation of the luciferase-expressing human bladder cancer UM-UC-3 cells, and the *in vivo* imaging of a luciferase activity was performed (prior to administration). Thereafter, 50 μl of MA-T was administered intravesically, and the *in vivo* imaging was performed again 2 days later. As a result, as will be described below, a remarkable decrease in a luciferase activity was observed by the MA-T administration.

[(5-1) Preparation of Bladder Cancer Cell Orthotopic Transplant Model]

ApGL4.51 [luc2/CMV/Neo] vector (Promega Corporation) was linearized with PstI (NEBs), and then the pGL4.51 linearization vector was purified using Wizard SV Gel and PCR Clean-Up System. Luciferase-expressing human bladder cancer UM-UC-3 Fluc cells were produced by transfecting pGL4.51 linearization vectors to UM-UC-3 cells by the forward method using Lipofectamine 3000 and selecting 3000 μg/mL G-418 (trade name, Wako). These cells were used to construct an orthotopic bladder cancer cell transplant model by the following procedure.

(1) 6 to 7-week old female BALB/c Slc-nu/nu was held in a supine position under isoflurane anesthesia, and then a catheter was inserted from a urethral orifice into a bladder using 24G3/4 THERFLOW F&F (trade name, Terumo) with the needle removed, to drain urine.
(2) 100 μL of 0.2% Trypsin (Life Technologies Corporation)/0.05% EDTA (DOJINDO) was injected intravesically. With the catheter inserted, the urethral orifice was closed with a dispo clamp (AM-1 30 g, Natsume Seisakusho Co., Ltd.) in a state where a catheter was inserted, and then the catheter was removed slowly.
(3) 5 minutes after Trypsin/EDTA injection, the mouse was placed prone from the supine, and Trypsin/EDTA was drained by abdominal abrasion.
(4) 100 μL of PBS was injected into the bladder, and the bladder was washed by repeating 2 to 3 times of the injecting and draining PBS.
(5) UM-UC-3 Fluc cells (5.0 × $10^6$ cells/mouse) were suspended in 50 μL of serum-free DMEM medium via a catheter and injected intravesically. Thereafter, the urethral orifice was closed with a clamp to prevent cell leakage, and the catheter was removed.
(6) The mouse was placed in an anesthesia box (Sinano) and left for 2 hours.
(7) 2 hours after the cell injection, the clamp was removed, and the mouse was returned to the original cage after the state of the mouse was checked.

*In vivo* imaging of UM-UC-3 Flue cell intrabladder-transplanted mouse was performed by the following procedure.

(1) As a substrate for luciferase, 4 mg of VivoGlo In Vivo Imaging Substrates (trade name, Promega Corporation) was dissolved in 200 μL of PBS. 200 μL of the prepared solution was administered intraperitoneally. The mouse was left in the cage for 3 minutes after administration and then was put under anesthesia for 3 minutes. Thereafter, images were taken at Pixel Bining 1 × 1 with an exposure time of 60 seconds using an imaging device NightOWL (trade name, BERTHOLD TECHNOLOGIES).

(2) Luciferase luminescence was measured on days 10 and 24 after transplantation, and the ratio was expressed as a multiple as the luminescence ratio.

In addition, MA-T was administered after *in vivo* imaging as described above. Specifically, 50 μl of 100 ppm MA-T was administered intravesically after luciferase activity determination 10 days after UM-UC-3 Flue transplantation. The mouse was allowed to play in the cage for 3 minutes after administration and was put under anesthesia for an additional 3 minutes. 2 days later, *in vivo* imaging was performed again by taking images at Pixel Bining 1 × 1 with an exposure time of 60 seconds using an imaging device NightOWL (trade name,

BERTHOLD TECHNOLOGIES).

The photograph of FIG. 5 shows the results of *in vivo* imaging before and 2 days after MA-T administration. In FIG. 5, the "MA-T107" represents a mouse to which MA-T was administered, and the "Control" represents a mouse to which 50μl of PBS was intravesically administered, instead of MA-T. As shown in FIG. 5, little luminescence reduction or tumor shrinkage was observed in the mouse to which no MA-T was administered, whereas tumor shrinkage was apparent in the mouse to which MA-T was administered.

[(5-2) Body Weight Change and Histopathological Analysis of Bladder of MA-T Intravesically Administered Mouse]

50 μl of 100 ppm MA-T or PBS as a Control was intravesically administered, twice a week for 2 weeks, to the mouse with which a bladder orthotopic transplant model was prepared in the same manner as described in "(5-1) Preparation of Bladder Cancer Cell Orthotopic Transplant Model". After administration, the body weight change of the mouse was measured until 10 days later. The graph of FIG. 6 shows the results. The horizontal axis indicates the number of days, and the vertical axis indicates the mouse body weight (body weight change rate) with the mouse body weight at the start of measurement (day 0) being 1. The "MA-T107" represents a mouse to which MA-T was administered, and the "PBS" represents a mouse to which PBS was administered as a Control. As shown in FIG. 6, no significant weight loss was observed with the MA-T administration.

In addition, the mouse bladder was excised 10 days after the end of administration, and the histopathological analysis thereof was carried out. The results are shown in the micrographs of FIG. 7. The micrographs on the upper side of FIG. 7 show an entire view of the bladder, and the micrographs on the lower side of FIG. 7 show an enlarged view. As shown in FIG. 7, no histopathological abnormalities were observed in the PBS-administered and MA-T-administered groups.

[(6) Tumor Histopathological Analysis]

The bladder of the UM-UC-3 Fluc- transplanted mice of "(5) Antitumor Effect of MA-T using Luciferase-Expressing Bladder Cancer Cell Orthotopic Transplant Model Mouse" on day 24 was excised and fixed with 10% neutral buffered formalin to prepare a paraffin block of tumor tissue, and hematoxylin-eosin (HE) staining was performed.

The graphs of FIG. 8 show the luciferase luminescence rate of bladder tumors measured on days 10 and 24 after the transplantation and the bladder weight measured on day 24 after the dissection. In FIG. 8, the "Control" represents a PBS sample (Control), and the "MA-T" represents a 100 ppm MA-T sample (i.e., a sample to which the MA-T epithelial cancer therapeutic agent of the present invention was added). The photographs on the right side of FIG. 8 are taken after HE staining. As shown in FIG. 8, it was verified that the proliferation of bladder cancer cells was inhibited and the bladder tumor was histologically damaged in the MA-T sample of the present invention as compared with the PBS sample (Control).

[(7) Evaluation on Antitumor Effect of MA-T Using Bladder Cancer Patient-Derived Xenograft (PDX) Mouse]

A mouse model to which a bladder cancer clinical specimen-derived tumor was passaged was used. Tumors were excised from three SHO Hairless SCID mice (male, 4-week old at tumor transplantation) whose tumors had been passaged (passage 8) 2 months earlier. The excised tumor was cut into approximately 5-mm squares, and the obtained tumor tissue sections were immersed in a 100 ppm MA-T aqueous solution or a 1 × PBS solution, as a control, for 120 minutes. The tumors were then transplanted subcutaneously to SHO Hairless SCID mice (male, 4-week old) (PBS-immersed group: n = 10, MA-T-immersed group: n = 10), and the tumor diameters (major and minor diameters) were measured every 3 days from the date of transplantation. The tumor volume was calculated by the following equation (A). Tumors were excised on day 32 after transplantation, and each tumor weight was measured. Statistical processing was performed by student's t-test.

$$\text{Tumor volume} = (\text{major axis} \times \text{minor axis}\ \text{^}2) / 2 \qquad (A)$$

[(7-1) Tumor Histopathological Analysis]

The tumor tissues excised from the PDX mice of "(7) Evaluation on Antitumor Effect of MA-T Using Bladder Cancer Patient-Derived Xenograft (PDX) Mouse" were fixed with 10% neutral buffered formalin to prepare a paraffin block of tumor tissue, and hematoxylin-eosin (HE) staining was performed.

The graph on the left side of FIG. 9 shows the relationship between the number of days after tumor transplantation and the tumor volume. The horizontal axis indicates the number of days after transplantation (day), and the vertical axis indicates the tumor volume ($mm^3$). The graph on the right side of FIG. 9 shows the weight (mg) of the tumor dissected and excised on day 32 after transplantation. In FIG. 9, the "Control" represents a PBS sample (Control), and the "MA-T" represents a 100 ppm MA-T sample (i.e., a sample to which the MA-T epithelial cancer therapeutic agent of the present invention was added). As shown in FIG. 9, it was verified that the proliferation of bladder cancer tumor was inhibited in the group treated with MA-T epithelial cancer therapeutic agent of the present invention as compared with the PBS sample (Control).

Furthermore, as shown in FIG. 10, as a result of the HE staining of the tumor excised on day 32, tumor necrotic images were histologically verified in a MA-T aqueous solution-treated group.

[Example 2: Bladder Cancer Therapeutic Agent Containing Sodium Chlorite ($NaClO_2$)]

A bladder cancer therapeutic agent containing $NaClO_2$ (an epithelial cancer therapeutic agent of the present invention) was produced as follows, and the effect thereof was verified. In the following description, the epithelial cancer therapeutic agent containing $NaClO_2$ of the present invention may be referred to as the "$NaClO_2$ epithelial cancer therapeutic agent of the present invention" or simply as the "$NaClO_2$ epithelial cancer therapeutic agent".

[(1) Cell Culture]

Bladder cancer cell line UM-UC-3 and colon cancer cell lines HT-29 and HCT116 were obtained from American Type Culture Collection (ATCC), bladder cancer cell line BOY-12E was obtained from Tohoku University, bladder cancer cell lines UM-UC-2, 5637, and EJ-1 were obtained from Nara Medical University. Uterine cancer cell line HeLa, cervical cancer cell line HeLaS3, tongue cancer cell line HSC-3, and oral cancer cell line HO-1-u-1 were obtained from the Japanese Collection of Research Bioresources (JCRB) cell bank.

UM-UC-3 cells, UM-UC-2 cells, EJ-1 cells, 5637 cells, and BOY-12E cells were cultured and used in the manner described in "(1) Cell Culture" of Example 1. HeLa cells, HeLaS3 cells, and HSC-3 cells were cultured at 37°C under 5% $CO_2$ using a medium, which was obtained by adding Fetal bovine serum (FBS, Biosera) to E-MEM (FUJIFILM Wako Pure Chemical Corporation) so as to become 10% by volume, HO-1-u-1 cells were cultured at 37°C under 5% $CO_2$ using a medium, which was obtained by adding Fetal bovine serum (FBS, Biosera) to Dulbecco's modifwied Eagle's medium-F12 medium (1: 1 mix) (FUJIFILM Wako Pure Chemical Corporation) so as to become 10% by volume, HT-29 cells and HCT116 cells were cultured at 37°C under 5% $CO_2$ using a medium, which was obtained by adding Fetal bovine serum (FBS, Biosera) to McCoy's 5a Medium (FUJIFILM Wako Pure Chemical Corporation) so as to become 10% by volume. When the cells were passaged, 0.025% Trypsin/EDTA (ethylenediaminetetraacetic acid) was added to the cells and incubated at 37°C under 5% $CO_2$ to detach the cells, and then centrifuged at 1500 rpm for 3 minutes to recover the cells.

[(2) Reagent Preparation]

1 × PBS aqueous solution of $NaClO_2$ was prepared by diluting a 10000 ppm $NaClO_2$ (sodium chlorite) aqueous solution (Lot. No. 170612C) with 10 × phosphate buffered saline (PBS) and distilled water (DW) so as to eventually became 1 × PBS as shown in the table below. These aqueous solutions correspond to the epithelial cancer therapeutic agent of the present invention containing $NaClO_2$, that is, the " $NaClO_2$ epithelial cancer therapeutic agent of the present invention". In the PBS, the "10×" represents a 10-fold concentration, and the "1×" represents a 1-fold concentration. Table 1 below shows the amount of each solution required when the total amount of the prepared $NaClO_2$ aqueous

solution is 400 μL.

[Table 2]

| Concentration [ppm] | 10000 ppm $NaClO_2$ aqueous solution [μL] | 10 × PBS [μL] | DW [μL] |
|---|---|---|---|
| 0 | 0 | 40 | 360 |
| 100 | 4 | 40 | 356 |
| 200 | 8 | 40 | 352 |
| 300 | 12 | 40 | 348 |
| 400 | 16 | 40 | 344 |
| 500 | 20 | 40 | 340 |
| 600 | 24 | 40 | 336 |
| 700 | 28 | 40 | 332 |
| 800 | 32 | 40 | 328 |
| 900 | 36 | 40 | 324 |
| 1000 | 40 | 40 | 320 |

[(3) WST-8 assay]

WST-8 assay was performed as follows.

[Study of Reaction Time and Concentration of $NaClO_2$ Aqueous Solution]

The above cells were seeded in a 96-well plate. At this time, UM-UC-3, UM-UC-2, EJ-1, and BOY-12E cells were seeded at 1200 cells/well, and 5637 cells were seeded at 1500 cells/well. On the next day, the media were removed from all the wells, and $NaClO_2$ aqueous solutions having a concentration of 500 ppm (reaction time study) and other concentrations were added to triplicate wells at 100 μL/well. The plates were incubated at 37°C under 5% $CO_2$ for 30, 60, 90, and 120 minutes (for reaction time study, the maximum time was set at 120 minutes, with consideration given to tolerable time for urination by intravesical instillation in clinical trials) and another 120 minutes (for concentration study), and then the solutions in the wells were removed. Subsequently, 90 μL of normal culture medium was added to all the wells, and the cells were cultured for additional 48 hours. Thereafter, 10 μL of Cell Counting Kit-8 (trade name, Dojindo Laboratories [DOJINDO]) was added per well, and incubated at 37°C under 5% $CO_2$ for 2 hours, and the absorbance at an absorbance wavelength of 450 nm and the absorbance at a control wavelength of 630 nm were measured using an iMark microplate reader (trade name, Bio-Rad Laboratories, Inc.).

FIG. 11 shows a graph (left graph) showing the cell proliferation rate when a 500 ppm $NaClO_2$ aqueous solution was contacted with UM-UC-3 cells for each period of time and a graph (right graph) showing the concentration-dependency of a $NaClO_2$ aqueous solution on cell proliferation of each bladder cancer cell. The horizontal axis indicates time (min) in the left graph, and the horizontal axis indicates concentration (ppm) in the right graph. The vertical axis indicates the cell proliferation rate expressed as a normalized index. As shown in FIG. 11, it was verified that the cell proliferation was inhibited in a time-dependent manner by incubation of 500 ppm $NaClO_2$ aqueous solution with UM-UC-3 cells, and the cell proliferation was inhibited more than 60% by 120 minutes incubation. In addition, it was verified that the concentrationdependent proliferation inhibition was observed in 120 minutes incubation of $NaClO_2$ aqueous solution for various bladder cancer cells, and that the bladder cancer proliferation was almost completely inhibited at 500 ppm or more.

FIG. 12 shows photomicrographs of UM-UC-3 cells immediately (0 h) after or 24 hours (24-hour-culture) after being incubated with PBS or $NaClO_2$ for 120 minutes and removed. Compared with the Control group (upper left photomicrograph), the $NaClO_2$ group (upper right photomicrograph) showed morphological changes in the cells 120 minutes after being incubated with $NaClO_2$ (0 hours after $NaClO_2$ removal). Then, the cells were further incubated with $NaClO_2$ for 120 minutes, and regression of cells was observed at 24 hours after removal (lower middle photomicrograph), and formation of a vesicle, which was considered to be an apoptotic vesicle, was observed in a partially magnified photomicrograph (lower right photomicrograph). The "Control" represents a PBS-added group sample to which no $NaClO_2$ was added (Control), and the "$NaClO_2$" represents a 500 ppm $NaClO_2$-added sample (i.e., a sample to which the $NaClO_2$ epithelial cancer therapeutic agent of the present invention was added).

[(4) Apoptosis Analysis]

UM-UC-3 cells were seeded in a six-well plate at $5\times 10^4$ cells/well. The culture medium was removed on the next day and a 500 ppm $NaClO_2$ aqueous solution was added. Thereafter, the resultants were incubated at 37°C under 5% $CO_2$ for 120 minutes. After the $NaClO_2$ aqueous solution was removed, the normal culture solution was added, and the cells were cultured for 24 hours, and then the cells including the supernatant were recovered and centrifuged at 3000 rpm for 5 minutes to obtain cell pellets. After the pellet was washed twice with 1 × PBS, 294 μL of 1 × Binding buffer, 3 μL of Propidium iodide, and 3 μL of Annexin V of Annexin V-FITC Apoptosis Detection Kit (trade name, Bio Vision) were added, and the pellets were suspended. The suspension was passed through a 37 μm nylon mesh (NBC MeshTech Inc.) and transferred to a round bottom tube (Falcon). Thereafter, the population of each of the stained cells was analyzed by FACS Calibur (trade name, Becton, Dickinson and Company). The single stained sample of Propidium iodide and the single stained sample of Annexin V were prepared for a FACS voltage control. As a Control, the apoptotic analysis was performed in the same manner using samples treated in the same manner, except that PBS containing no $NaClO_2$ was used, instead of the $NaClO_2$ aqueous solution.

FIG. 13 shows the apoptotic analysis of UM-UC-3 cells. In FIG. 13, the "Control" represents a PBS sample (control) to which no $NaClO_2$ was added, and the "$NaClO_2$" represents a sample to which 500 ppm $NaClO_2$ was added (i.e., a sample to which the $NaClO_2$ epithelial cancer therapeutic agent of the present invention was added). As shown FIG. 13, the apoptotic rate of UM-UC-3 cells was 14.6% in the PBS sample to which no $NaClO_2$ was added (Control), whereas the apoptotic rate of UM-UC-3 cells was 43.8% in 500 ppm $NaClO_2$ to which the epithelial cancer therapeutic agent of the present invention was added. That is, it was verified that the $NaClO_2$ epithelial cancer therapeutic agent of the present invention had an effect of significantly inducing apoptosis in bladder cancer cells.

[(5) Cell Cycle Analysis]

UM-UC-3 cells were subjected to the same procedure as in [(4) Apoptosis Analysis] described above until the cells were pelleted. To the obtained cell pellet, 700 μL of a 70% aqueous ethanol solution was added one drop at a time and stored at -20°C overnight. The resultant was centrifuged at 3000 rpm for 5 minutes at 4°C, and then the supernatant was removed, and the resultant was washed twice with 1 × PBS. Further, 10 μL of 10 mg/mL RNase (trade name, FUJIFII,M Wako Pure Chemical Corporation) and 50 μL of 1 × PBS were added, and the resultant was incubated at room temperature for 30 minutes. Then, the resultant was suspended in 500 μL of FACS buffer (3% FCS in 1 × PBS) to which 50 μL of 0.5 mg/mL propidium iodide (PI, Sigma-Aldrich Co. LLC) solution was added. The suspension was passed through a 37 μm nylon mesh and then measured by FACS Calibur (Becton, Dickinson and Company). As a Control, a cell cycle analysis was performed in the same manner using a sample treated in the same manner except that PBS containing no $NaClO_2$ was used, instead of the $NaClO_2$ aqueous solution.

FIG. 14 shows the results of the cell cycle analysis of UM-UC-3 cells after 24 hours (overnight) of culturing after removing the $NaClO_2$ aqueous solution. In FIG. 14, the "Control" represents a PBS sample to which no $NaClO_2$ was added (Control), and the "$NaClO_2$" represents a sample to which 500 ppm $NaClO_2$ was added (i.e., a sample to which the $NaClO_2$ epithelial cancer therapeutic agent of the present invention was added). As shown in FIG. 14, the fraction ratio in subG1 phase indicating the apoptotic cell fraction was significantly increased in the group to which the $NaClO_2$ epithelial cancer therapeutic agent of the present invention was added as compared with the PBS group (Control). That is, it was verified that the $NaClO_2$ epithelial cancer therapeutic agent of the present invention induces apoptotic cell death in bladder cancer cells.

[(6) Evaluation on Antitumor Effect of $NaClO_2$ Epithelial Cancer Therapeutic Agent Using Bladder Cancer Patient-Derived Xenograft (PDX) Mouse]

The Evaluation was performed, using a mouse tumor model to which a bladder cancer clinical specimen-derived tumor was passaged, in the same manner as in "(7) Evaluation on Antitumor Effect of MA-T Using Bladder Cancer Patient-Derived Xenograft (PDX) Mouse" of Example 1, except that a 500 ppm $NaClO_2$ aqueous solution was used, instead of a 100 ppm MA-T aqueous solution. SHO Hairless SCID mice (male, 4-week old) used were n=8 in both the PBS-immersed group and $NaClO_2$-immersed group. The tumor diameters (major and minor diameters) were measured every 3 days from day 12 after the transplantation. The tumors excised on day 27 were photographed and weighed.

In the graph on the lower left of FIG. 15, the horizontal axis indicates the number of days from 12 days after the tumor transplantation, and the vertical axis indicates the tumor volume ($mm^3$). In addition, the photograph on the upper side of FIG. 15 shows the photograph of the tumor dissected and excised on day 27 after transplantation, and the graph on the lower right shows the weight (mg) of the excised tumor. In FIG. 15, the "Control" represents a PBS sample (Control), and the "$NaClO_2$" represents the tumor-transplantation results immersed in 500 ppm $NaClO_2$ (i.e., the $NaClO_2$ epithelial cancer therapeutic agent of the present invention). As shown in FIG. 15, it was verified that the

proliferation of bladder cancer tumors was significantly inhibited in the group immersed in the $NaClO_2$ epithelial cancer therapeutic agent of the present invention as compared with that of the PBS group (Control). In addition, from the photograph of the excised tumor and the analysis of the tumor weight, it was verified that the increase in bladder tumors was inhibited.

[(7) Verification of Radical Generation]

The radical productions of a $NaClO_2$ aqueous solution, a benzethonium chloride aqueous solution (radical generating catalyst), and MA-T were verified. UM-UC-3 cells were seeded in a 96-well plate at 5000 cells/well. On the next day, the medium was removed, and 50 μL of CellROX (trade name, Thermo Fisher Scientific, Inc.), which is a reagent for detection and quantification of reactive oxygen species in living cells diluted with Hank's Balanced Salt Solution (HBSS), was added at a concentration of 20 μM per well and incubated at 37°C for 1 hour. Then, the $NaClO_2$ aqueous solution (A), the benzethonium chloride aqueous solution (B), or MA-T, which were diluted with HBSS, were added at 50 μL/well, and the fluorescent intensity was measured over the time using a fluorescent plate reader GloMAX (trade name, Promega Corporation). The fluorescence of UM-UC-3 cells to which a $NaClO_2$ aqueous solution was added at a final concentration of 500 ppm was detected using BioZero fluorescence microscopy (KEYENCE CORPORATION.) with Excitation Filter (Red 627 nm) and Emission Filter (660 to 720 nm).

In the graph on the left side of FIG. 16, the vertical axis indicates the fluorescence intensity, and the horizontal axis indicates the sample to which $NaClO_2$, benzethonium chloride, or MA-T was added. It was verified that, between $NaClO_2$ (A) and benzethonium chloride (B), which alone were not capable of producing radicals, $NaClO_2$ (A) produced reactive acid species in a time-dependent manner by contacting the cells. Further, with 100 ppm $NaClO_2$ (A100) reactive oxygen species equivalent to or more than that with MA-T (a combination of 100 ppm $NaClO_2$ and 100 ppm benzethonium chloride) were detected, and it was verified that 500 ppm $NaClO_2$ alone, which has been verified to have an effect on bladder cancer cells, showed remarkable reactive oxygen production. This suggests that reactive oxygen species different from that of MA-T were produced by contacting $NaClO_2$ with bladder cancer cells. The photographs on the right side of FIG. 16 show images of the cells, taken by BioZero fluorescent microscopy. The upper photographs are images of the Control to which no $NaClO_2$ or benzethonium chloride was added, and the lower photographs are images of the sample to which the $NaClO_2$ aqueous solution was added at a final concentration of 500 ppm. As shown in FIG. 16, the fluorescence showing remarkable active oxygen production in bladder cancer cells were observed in a sample with 500 ppm $NaClO_2$ alone.

[(8) Effects of $NaClO_2$ on Tongue Cancer Cells, Oral Cancer Cells, Uterine Cancer Cells, Cervical Cancer Cells, and Colon Cancer Cells]

The effect of the $NaClO_2$ epithelial cancer therapeutic agent of the present invention as a therapeutic agent for epithelial cancer cells other than bladder cancer cells was verified as follows.

A cancer cell proliferation assay was performed by culturing the respective cancer cells for 48 hours in the same manner as in [(3) WST-8 assay], except that the incubation time was changed to 60 minutes (1 hour) instead of 120 minutes, in consideration of the acceptable time for the target cancer patient, and that the bladder cancer cells were replaced with tongue cancer cells "HSC-3", oral cancer cells "HO-1-u-1", uterine cancer cells "HeLa", cervical cancer cells "HeLaS3", colon cancer cells "HT29", or colon cancer cells "HT116". The graphs of FIGs. 17, 18, and 19 show the viability of each cancer cells after 48 hours of culturing. In the graphs of FIGs. 17, 18, and 19, the horizontal axis indicates the concentration (ppm) of the $NaClO_2$ aqueous solution, and the vertical axis indicates the viability (%) of the cancer cells.

As shown in FIGs. 17, 18 and 19, it was verified that the $NaClO_2$ epithelial cancer therapeutic agent according to the present invention showed the effect of inhibiting proliferation of cancer cells, even with respect to tongue cancer cells, oral cancer cells, uterine cancer cells, cervical cancer cells, and colorectal cancer cells.

[(9) Verification of Effect of Radical Generating Catalyst]

The uterine cancer cells "HeLa" and "HeLaS3" were cultured for 48 hours and the cancer cell proliferation assay was performed in the same manner as in "(8) Effects of $NaClO_2$ on Tongue Cancer Cells, Oral Cancer Cells, Uterine Cancer Cells, Cervical Cancer Cells, and Colon Cancer Cells" except that benzethonium chloride (radical generating catalyst) was added to the $NaClO_2$ aqueous solution. The concentration of benzethonium chloride in the $NaClO_2$ aqueous solution was varied to be 5 ppm, 10 ppm, 15 ppm, or 20 ppm, respectively, and the same assay was performed for each concentration.

The results are shown in the graphs of FIGs. 20 and 21. In FIGs. 20 and 21, the "agent A" represents $NaClO_2$, and the "agent B" represents benzethonium chloride (radical generating catalyst). In FIGs. 20 and 21, the horizontal axis indicates the concentration (ppm) of the $NaClO_2$ aqueous solution, and the vertical axis indicates the viability (%)

of the cancer cells. In FIGs. 20 and 21, the "Control" indicates the case where no benzethonium chloride was added, i.e., the result of "(9) Effects of $NaClO_2$ on Tongue Cancer Cells, Oral Cancer Cells, Uterine Cancer Cells, Cervical Cancer Cells, and Colon Cancer Cells". As shown in FIGs. 20 and 18, while the effect of inhibiting the cancer cell proliferation was observed even with $NaClO_2$ alone, the additive effect of inhibiting the cancer cell proliferation was observed by adding benzethonium chloride (radical generating catalyst).

[Example 3: Pancreatic Cancer Therapeutic Agent Containing Sodium Chlorite ($NaClO_2$)]

A pancreatic cancer therapeutic agent containing $NaClO_2$ (an epithelial cancer therapeutic agent of the present invention) was produced as follows, and the effect thereof was verified. The pancreatic cancer therapeutic agent of the present example corresponds to the epithelial cancer therapeutic agent containing $NaClO_2$ of the present invention, i.e., the "$NaClO_2$ epithelial cancer therapeutic agent of the present invention" or simply the "$NaClO_2$ epithelial cancer therapeutic agent".

The effect of the $NaClO_2$ aqueous solution ($NaClO_2$ epithelial cancer therapeutic agent of the present invention) on the primary cultured pancreatic cancer cells was analyzed as follows.

[(1) Cell Culture]

Primary cultured cells PK05 and PK10 established at Graduate School and School of Pharmaceutical Sciences, Osaka University from pancreatic cancer specimens were used. These cells were cultured at 37°C under 5% $CO_2$ using a medium, which was obtained by adding FBS (Biosera) to RPMI-1640 (trade name, produced by FUJIFII,M Wako Pure Chemical Corporation) so as to become 10% by volume. When the cells were passaged, 0.025% Trypsin/EDTA (ethylenediaminetetraacetic acid) was added to the cells, and the resultant was incubated at 37°C under 5% $CO_2$ to detach the cells, and then centrifuged at 1500 rpm for 3 minutes to recover the cells.

[(2) Reagent Preparation]

In the same manner as in "(2) Reagent Preparation" of "Example 2: Bladder Cancer Therapeutic Agent containing sodium chlorite ($NaClO_2$)", a 10000 ppm $NaClO_2$ (sodium chlorite) aqueous solution (Lot. No. 170612C), 10 × phosphate buffered saline (PBS), and distilled water (DW) were mixed so as to eventually became 1 × PBS having a predetermined concentration of $NaClO_2$. Thereby, 1 × PBS aqueous solutions containing $NaClO_2$ at concentrations of 0 ppm, 2 ppm, 4 ppm, 8 ppm, 16 ppm, 31 ppm, 63 ppm, 125 ppm, 250 ppm, or 500 ppm were prepared. These aqueous solutions are the same as the aqueous solutions prepared in "(2) Reagent Preparation" of "Example 2: Bladder Cancer Therapeutic Agent containing sodium chlorite ($NaClO_2$)" except for the $NaClO_2$ concentration. These aqueous solutions correspond to the epithelial cancer therapeutic agent of the present invention containing $NaClO_2$, i.e., the "$NaClO_2$ epithelial cancer therapeutic agent of the present invention". In the PBS, the "10×" represents a 10-fold concentration, and the "1×" represents a 1-fold concentration.

[(3) Cell Viability Evaluation]

PK05 cells or PK10 cells cultured in "(1) Cell Culture" were seeded in a 96-well plate at 5000 cells/well. The medium was removed from all the wells on the next day, and the $NaClO_2$ aqueous solutions of different concentrations prepared in "(2) Reagent Preparation" were added to triplicate wells at 100 μL/well. The plates were cultured at 37°C under 5% $CO_2$ for 48 hours. Thereafter, 10 μL of Cell Counting Kit-8 (trade name, Dojindo Laboratories [DOJINDO]) was added per well, and the resultant was incubated at 37°C under 5% $CO_2$ for 2 hours, and the absorbance at an absorbance was measured with wavelength of 450 nm and with a control wavelength of 630 nm using an iMark microplate reader (trade name, Bio-Rad Laboratories, Inc.). The results are shown in graphs of FIG. 22. In FIG. 22, the "agent A" represents $NaClO_2$. In the graphs of FIG. 22, the horizontal axis indicates the concentration (ppm) of the $NaClO_2$ aqueous solution, and the vertical axis indicates the viability (%) of the cancer cells. In the vertical axis, the viability (%) of cancer cells was expressed with the cell viability in the wells as that of the 1×PBS aqueous solution (Control) having a $NaClO_2$ concentration of 0 ppm (i.e., containing no sodium chlorite) as 100%. As shown in FIG. 22, a $NaClO_2$ concentrationdependent proliferation inhibitory effect was verified on both the pancreatic cancer cells PK05 and PK10. That is, it was verified that the $NaClO_2$ aqueous solution prepared in "(2) Reagent Preparation" serves as a pancreatic cancer therapeutic agent (i.e., the epithelial cancer therapeutic agent). In addition, it was verified that the pancreatic cancer cell proliferation was almost completely inhibited by incubation with a $NaClO_2$ aqueous solution having a concentration of 31 ppm or more.

(Supplementary Notes)

Some or all of the above embodiments and examples may be described as in the following Supplementary Notes, but are not limited thereto.

(Supplementary Note 1)

An epithelial cancer therapeutic agent, including:

at least one selected from the group consisting of oxo acid, oxo acid ion, and oxo acid salt, wherein
the epithelial cancer therapeutic agent is used for treatment or prevention of epithelial cancer.

(Supplementary Note 2)

The epithelial cancer therapeutic agent according to Supplementary Note 1, wherein
the epithelial cancer is a cancer of at least one tissue cancer selected from the group consisting of a urinary system, a reproductive system, a digestive system, a circulatory system, and a respiratory system.

(Supplementary Note 3)

The epithelial cancer therapeutic agent according to Supplementary Note 1 or 2, wherein
the epithelial cancer is at least one selected from the group consisting of bladder cancer, uterine cancer, cervical cancer, ovarian cancer, fallopian tube cancer, testicular cancer, prostate cancer, colon cancer, small intestine cancer, duodenal cancer, gastric cancer, esophageal cancer, laryngeal cancer, oral cavity cancer, tongue cancer, pharyngeal cancer, liver cancer, pancreatic cancer, gallbladder cancer, spleen cancer, peritoneal cancer, lung cancer, and eye cancer.

(Supplementary Note 4)

The epithelial cancer therapeutic agent according to any one of Supplementary Notes 1 to 3, wherein
the oxo acid is a halogen oxo acid.

(Supplementary Note 5)

The epithelial cancer therapeutic agent according to Supplementary Note 4, wherein
the halogen oxo acid is at least one selected from the group consisting of chlorine oxo acid, bromine oxo acid, and iodine oxo acid.

(Supplementary Note 6)

The epithelial cancer therapeutic agent according to Supplementary Note 4 or 5, wherein
the halogen oxo acid is at least one selected from the group consisting of hypohalous acid, halous acid, halogen acid, and perhalogen acid.

(Supplementary Note 7)

The epithelial cancer therapeutic agent according to Supplementary Note 4 or 5, wherein
the halogen oxo acid is at least one selected from the group consisting of hypochlorous acid, chlorous acid, chloric acid, perchloric acid, hypobromous acid, bromous acid, bromic acid, perbromic acid, hypoiodous acid, iodous acid, iodic acid, andperiodic acid.

(Supplementary Note 8)

The epithelial cancer therapeutic agent according to any one of Supplementary Notes 1 to 7, further comprising:

a radical generating catalyst, wherein
the radical generating catalyst is a substance that catalyzes radical generation from at least one substance selected from the group consisting of the oxo acid, the oxo acid ion, and the oxo acid salt, contained in the epithelial cancer therapeutic agent.

(Supplementary Note 9)

The epithelial cancer therapeutic agent according to Supplementary Note 8, wherein the radical generating catalyst comprises at least one material selected from the group consisting of ammonium, amino acids, proteins, peptides, phospholipids, and salts thereof.

(Supplementary Note 10)

The epithelial cancer therapeutic agent according to Supplementary Note 9, wherein

the ammonium is an ammonium salt represented by the following chemical formula (XI):

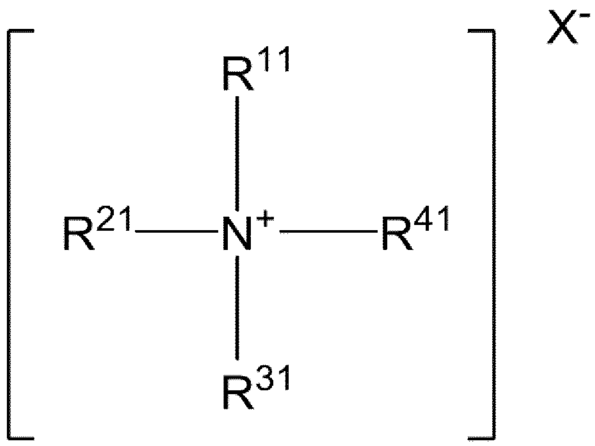

(XI)

where in the chemical formula (XI),
$R^{11}$, $R^{21}$, $R^{31}$, and $R^{41}$ are each a hydrogen atom, or an aromatic ring, or an alkyl group, the alkyl group may comprise an ether bond, a carbonyl group, an ester bond, or an amide bond, or an aromatic ring,
$R^{11}$, $R^{21}$, $R^{31}$, and $R^{41}$ may be identical to or different from each other,
two or more of $R^{11}$, $R^{21}$, $R^{31}$, and $R^{41}$ may be integrated to form a ring structure with $N^+$ to which they are bonded, the ring structure may be saturated or unsaturated, aromatic or non-aromatic, and may or may not have one or more substituents, and
$X^-$ is an anion.

(Supplementary Note 11)

The epithelial cancer therapeutic agent according to Supplementary Note 10, wherein

the ammonium salt represented by the chemical formula (XI) is an ammonium salt represented by the following chemical formula (XII):

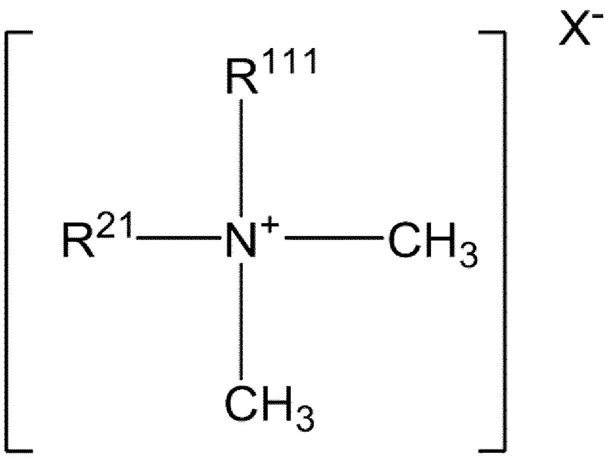

(XII)

where in the chemical formula (XII),
$R^{111}$ is an alkyl group having 5 to 40 carbon atoms, and may comprise an ether bond, ketone (carbonyl group), an ester bond, or an amide bond, a substituent, or an aromatic ring, and
$R^{21}$ and $X^-$ are the same as those in the chemical formula (XI).

(Supplementary Note 12)

The epithelial cancer therapeutic agent according to any one of Supplementary Notes 9 to 11, wherein

the ammonium is at least one selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxytropium, carbachol, glycopyrronium, safranin, sinapine, tetraethylammonium bromide,
hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, betalain, lecithin, adenine, guanine, cytosine, thymine, uracil, and cholines.

(Supplementary Note 13)

The epithelial cancer therapeutic agent according to Supplementary Note 10, wherein

the ammonium salt represented by the chemical formula (XI) is an ammonium salt represented by the following chemical formula (XIV):

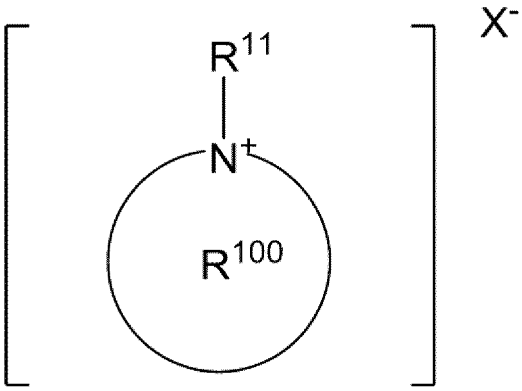

(XIV)

where in the chemical formula (XIV),
$R^{100}$ may form a ring structure, which may be saturated or unsaturated, aromatic or non-aromatic, and may or may not have one or more substituents, and
$R^{11}$ and $X^-$ are the same as those in the chemical formula (XI).

(Supplementary Note 14)

The epithelial cancer therapeutic agent according to any one of Supplementary Notes 8 to 13, wherein the radical generating catalyst has a Lewis acidity of 0.4 eV or more.

(Supplementary Note 15)

The epithelial cancer therapeutic agent according to any one of Supplementary Notes 1 to 14, which is an epithelial cancer cell apoptosis promoting agent.

(Supplementary Note 16)

A pharmaceutical composition, compsiring:

at least one selected from the group consisting of oxo acid, oxo acid ion, and oxo acid salt, wherein
the pharmaceutical composition is used for treatment or prevention of epithelial cancer.

(Supplementary Note 17)

The pharmaceutical composition according to Supplementary Note 16, wherein
the epithelial cancer is a cancer of at least one system selected from the group consisting of a urinary system, a reproductive system, a digestive system, a circulatory system, and a respiratory system.

(Supplementary Note 18)

The pharmaceutical composition according to Supplementary Note 16 or 17, wherein
the epithelial cancer is at least one system cancer selected from the group consisting of bladder cancer, uterine cancer, cervical cancer, ovarian cancer, fallopian tube cancer, testicular cancer, prostate cancer, colon cancer, small intestine cancer, duodenal cancer, gastric cancer, esophageal cancer, laryngeal cancer, oral cavity cancer, tongue cancer, pharyngeal cancer, liver cancer, pancreatic cancer, gallbladder cancer, spleen cancer, peritoneal cancer, lung cancer, and eye cancer.

(Supplementary Note 19)

The pharmaceutical composition according to any one of Supplementary Notes 16 to 18, wherein
the oxo acid is a halogen oxo acid.

(Supplementary Note 20)

The pharmaceutical composition according to Supplementary Note 19, wherein the halogen oxo acid is at least one selected from the group consisting of chlorine oxo acid, bromine oxo acid, and iodine oxo acid.

(Supplementary Note 21)

The pharmaceutical composition according to Supplementary Note 19 or 20, wherein the halogen oxo acid is at least one selected from the group consisting of hypohalous acid, halous acid, halogen acid, and perhalogen acid.

(Supplementary Note 22)

The pharmaceutical composition according to Supplementary Note 19 or 20, wherein the halogen oxo acid is at least one selected from the group consisting of hypochlorous acid, chlorous acid, chloric acid, perchloric acid, hypobromous acid, bromous acid, bromic acid, perbromic acid, hypoiodous acid, iodous acid, iodic acid, andperiodic acid.

(Supplementary Note 23)

The pharmaceutical composition according to any one of Supplementary Notes 26 to 22, further comprising:

a radical generating catalyst, wherein the radical generating catalyst is a substance that catalyzes radical generation from at least one substance selected from the group consisting of the oxo acid, the oxo acid ion, and the oxo acid salt, contained in the pharmaceutical composition.

(Supplementary Note 24)

The pharmaceutical composition according to Supplementary Note 23, wherein the radical generating catalyst comprises at least one material selected from the group consisting of ammonium, amino acids, proteins, peptides, phospholipids, and salts thereof.

(Supplementary Note 25)

The pharmaceutical composition according to Supplementary Note 24, wherein

the ammonium is an ammonium salt represented by the following chemical formula (XI):

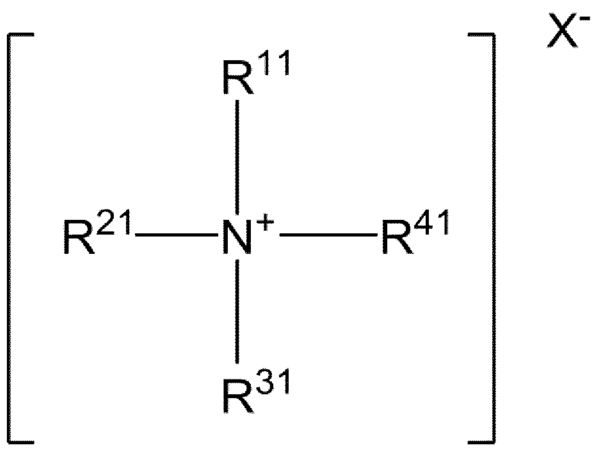

(XI)

where in the chemical formula (XI),
$R^{11}$, $R^{21}$, $R^{31}$, and $R^{41}$ are each a hydrogen atom or an aromatic ring, or an alkyl group, the alkyl group may comprise an ether bond, a carbonyl group, an ester bond, or an amide bond, or an aromatic ring,
$R^{11}$, $R^{21}$, $R^{31}$, and $R^{41}$ may be identical to or different from each other,
two or more of $R^{11}$, $R^{21}$, $R^{31}$, and $R^{41}$ may be integrated to form a ring structure with $N^+$ to which they are bonded, the ring structure may be saturated or unsaturated, aromatic or non-aromatic, and may or may not have one or more substituents, and
$X^-$ is an anion.

(Supplementary Note 26)

The pharmaceutical composition according to Supplementary Note 25, wherein

the ammonium salt represented by the chemical formula (XI) is an ammonium salt represented by the following chemical formula (XII):

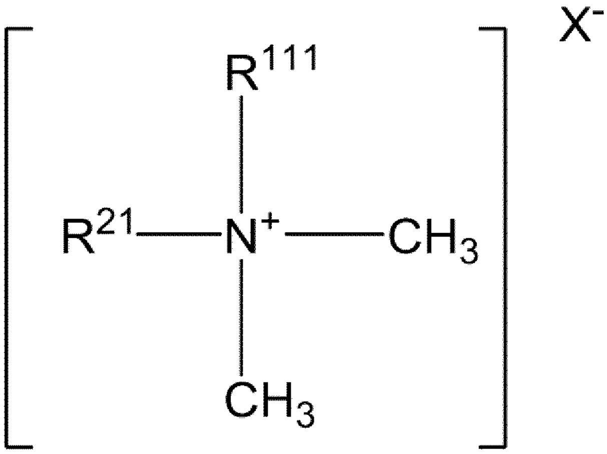

(XII)

where in the chemical formula (XII),
$R^{111}$ is an alkyl group having 5 to 40 carbon atoms, and may comprise an ether bond, ketone (carbonyl group), an ester bond, or an amide bond, a substituent, or an aromatic ring, and
$R^{21}$ and $X^-$ are the same as those in the chemical formula (XI).

(Supplementary Note 27)

The pharmaceutical composition according to any one of Supplementary Notes 24 to 26, wherein
the ammonium is at least one selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, betalain, lecithin, adenine, guanine, cytosine, thymine, uracil, and cholines.

(Supplementary Note 28)

The pharmaceutical composition according to Supplementary Note 25, wherein

the ammonium salt represented by the chemical formula (XI) is an ammonium salt represented by the following chemical formula (XIV):

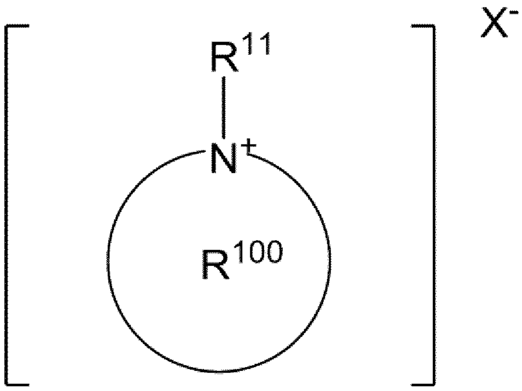

(XIV)

where in the chemical formula (XIV),
$R^{100}$ may form a ring structure, which may be saturated or unsaturated, aromatic or non-aromatic, and may or may not have one or more substituents, and
$R^{11}$ and $X^-$ are the same as those in the chemical formula (XI).

(Supplementary Note 29)

The pharmaceutical composition according to any one of Supplementary Notes 23 to 28, wherein the radical generating catalyst has a Lewis acidity of 0.4 eV or more.

(Supplementary Note 30)

The pharmaceutical composition according to any one of Supplementary Notes 16 to 29, which is an epithelial cancer cell apoptosis promoting agent.

(Supplementary Note 31)

Use of at least one selected from the group consisting of oxo acid, oxo acid ion, and oxo acid salt for producing the epithelial cancer therapeutic agent according to any one of Supplementary Notes 1 to 15.

(Supplementary Note 32)

Use of at least one selected from the group consisting of oxo acid, oxo acid ion, and oxo acid salt for producing the pharmaceutical composition according to any one of Supplementary Notes 16 to 30.

(Supplementary Note 33)

Use of at least one substance selected from the group consisting of oxo acid, oxo acid ion, and oxo acid salt as the epithelial cancer therapeutic agent according to any one of Supplementary Notes 1 to 15 or the pharmaceutical composition according to any one of Supplementary Notes 16 to 30.

(Supplementary Note 34)

A method for treating or preventing an epithelial cancer in a patient, comprising:
administering to the patient the epithelial cancer therapeutic agent according to any one of Supplementary Notes 1 to 15 or the pharmaceutical composition according to any one of Supplementary Notes 16 to 30.

(Supplementary Note 35)

A method for treating or preventing an epithelial cancer in a patient, comprising:
administering to the patient at least one substance selected from the group consisting of oxo acid, oxo acid ion, and oxo

acid salt.

(Supplementary Note 36)

Use of the epithelial cancer therapeutic agent according to any one of Supplementary Notes 1 to 15 or the pharmaceutical composition according to any one of Supplementary Notes 16 to 30, comprising:
administering to a patient the epithelial cancer therapeutic agent according to any one of Supplementary Notes 1 to 15 or the pharmaceutical composition according to any one of Supplementary Notes 16 to 30 to treat or prevent an epithelial cancer in the patient.

(Supplementary Note 37)

Use of at least one substance selected from the group consisting of oxo acid, oxo acid ion, and oxo acid salt comprising:
administering to a patient at least one substance selected from the group consisting of oxo acid, oxo acid ion, and oxo acid salt to treat or prevent an epithelial cancer in the patient.

While the present invention has been described above with reference to illustrative embodiments and examples, the present invention is by no means limited thereto. Various changes and variations that may become apparent to those skilled in the art may be made in the configuration and specifics of the present invention without departing from the scope of the present invention.

As described above, according to the present invention, it is possible to provide an epithelial cancer therapeutic agent with less side effects. The epithelial cancer therapeutic agent of the present invention is useful as a therapeutic agent for a wide variety of epithelial cancers such as bladder cancer, tongue cancer, oral cavity cancer, uterine cancer, cervical cancer, and colon cancer. The epithelial cancer therapeutic agent of the present invention is useful as an apoptosis-inducing epithelial cancer therapeutic agent by reactive oxygen production, for example, as described in the Examples.

This application claims priority from Japanese Patent Application No. 2020-089253 filed on May 21, 2020. The entire subject matter of the Japanese Patent Applications is incorporated herein by reference.

## Claims

**1.** An epithelial cancer therapeutic agent, comprising:

at least one material selected from the group consisting of oxo acid, oxo acid ion, and oxo acid salt, wherein the epithelial cancer therapeutic agent treats or prevents epithelial cancer.

**2.** The epithelial cancer therapeutic agent according to claim 1, wherein
the epithelial cancer is a cancer of at least one system selected from the group consisting of a urinary system, a reproductive system, a digestive system, a circulatory system, and a respiratory system.

**3.** The epithelial cancer therapeutic agent according to claim 1 or 2, wherein
the epithelial cancer is at least one tissue cancer selected from the group consisting of bladder cancer, uterine cancer, cervical cancer, ovarian cancer, fallopian tube cancer, testicular cancer, prostate cancer, colon cancer, small intestine cancer, duodenal cancer, gastric cancer, esophageal cancer, laryngeal cancer, oral cavity cancer, tongue cancer, pharyngeal cancer, liver cancer, pancreatic cancer, gallbladder cancer, spleen cancer, peritoneal cancer, lung cancer, and eye cancer.

**4.** The epithelial cancer therapeutic agent according to any one of claims 1 to 3, wherein
the oxo acid is a halogen oxo acid.

**5.** The epithelial cancer therapeutic agent according to claim 4, wherein
the halogen oxo acid is at least one material selected from the group consisting of chlorine oxo acid, bromine oxo acid, and iodine oxo acid.

**6.** The epithelial cancer therapeutic agent according to claim 4 or 5, wherein
the halogen oxo acid is at least one material selected from the group consisting of hypohalous acid, halous acid, halogen acid, and perhalogen acid.

**7.** The epithelial cancer therapeutic agent according to claim 4 or 5, wherein
the halogen oxo acid is at least one material selected from the group consisting of hypochlorous acid, chlorous acid, chloric acid, perchloric acid, hypobromous acid, bromous acid, bromic acid, perbromic acid, hypoiodous acid, iodous acid, iodic acid, and periodic acid.

**8.** The epithelial cancer therapeutic agent according to any one of claims 1 to 7, further comprising:

a radical generating catalyst, wherein
the radical generating catalyst is a substance that catalyzes radical generation from at least one substance selected from the group consisting of the oxo acid, the oxo acid ion, and the oxo acid salt, contained in the epithelial cancer therapeutic agent.

**9.** The epithelial cancer therapeutic agent according to claim 8, wherein
the radical generating catalyst comprises at least one material selected from the group consisting of ammonium, amino acids, proteins, peptides, phospholipids, and salts thereof.

**10.** The epithelial cancer therapeutic agent according to claim 9, wherein

the ammonium is an ammonium salt represented by following chemical formula (XI):

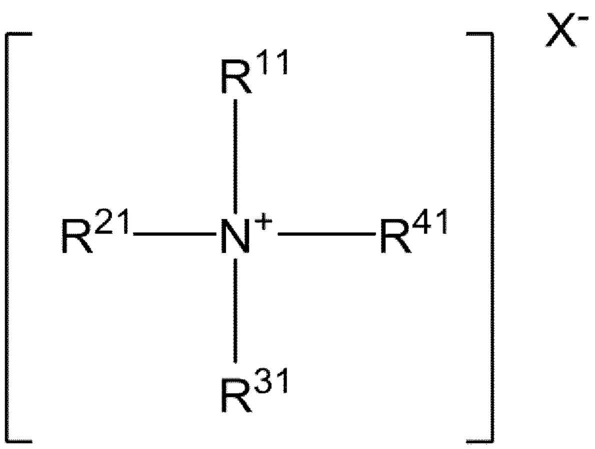

(XI)

where in the chemical formula (XI),
$R^{11}$, $R^{21}$, $R^{31}$, and $R^{41}$ are each a hydrogen atom or an aromatic ring, or an alkyl group, the alkyl group optionally comprises an ether bond, a carbonyl group, an ester bond, or an amide bond, or an aromatic ring,
$R^{11}$, $R^{21}$, $R^{31}$, and $R^{41}$are identical to or different from each other,
two or more groups of $R^{11}$, $R^{21}$, $R^{31}$, and $R^{41}$optionally are integrated to form a ring structure with $N^+$ to which the two or more groups are bonded, and the ring structure is saturated or unsaturated and aromatic or non-aromatic, and optionally have one or more substituents, and
$X^-$ is an anion.

**11.** The epithelial cancer therapeutic agent according to claim 10, wherein

the ammonium salt represented by the chemical formula (XI) is an ammonium salt represented by following chemical formula (XII):

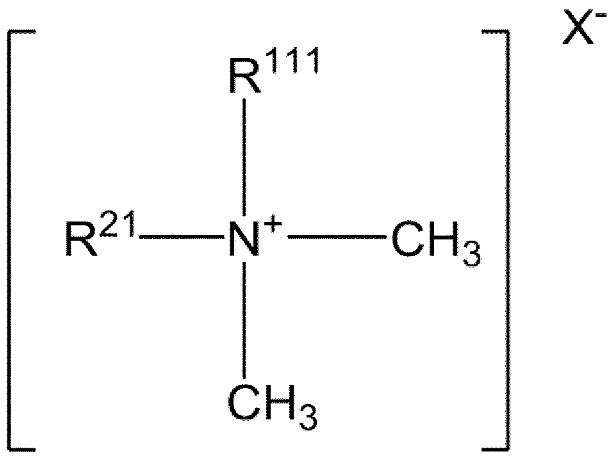

(XII)

where in the chemical formula (XII),
$R^{111}$ is an alkyl group having 5 to 40 carbon atoms, and optionally comprises an ether bond, ketone (carbonyl group), an ester bond, or an amide bond, a substituent, or an aromatic ring, and
$R^{21}$ and $X^-$ are same as the $R^{21}$ and the $X^-$ in the chemical formula (XI), respectively.

**12.** The epithelial cancer therapeutic agent according to any one of claims 9 to 11, wherein
the ammonium is at least one selected from the group consisting of benzethonium chloride, benzalkonium chloride, hexadecyltrimethylammonium chloride, tetramethylammonium chloride, ammonium chloride, methylammonium chloride, tetrabutylammonium chloride, cetylpyridinium chloride, hexadecyltrimethylammonium bromide, dequalinium chloride, edrophonium, didecyldimethylammonium chloride, benzyltriethylammonium chloride, oxytropium, carbachol, glycopyrronium, safranin, sinapine, tetraethylammonium bromide, hexadecyltrimethylammonium bromide, suxamethonium, sphingomyelin, ganglioside GM1, denatonium, trigonelline, neostigmine, paraquat, pyridostigmine, phellodendrine, pralidoxime methiodide, betaine, betanin, bethanechol, betalain, lecithin, adenine, guanine, cytosine, thymine, uracil, and cholines.

**13.** The epithelial cancer therapeutic agent according to claim 10, wherein

the ammonium salt represented by the chemical formula (XI) is an ammonium salt represented by following chemical formula (XIV):

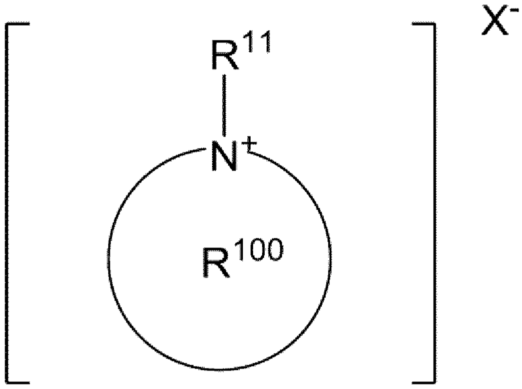

(XIV)

where in the chemical formula (XIV),
$R^{100}$ optionally forms a ring structure, which is saturated or unsaturated, aromatic or non-aromatic, and optionally

have one or more substituents, and
$R^{11}$ and $X^-$ are the same as the $R^{11}$ and the $X^-$ in the chemical formula (XI), respectively.

**14.** The epithelial cancer therapeutic agent according to any one of claims 8 to 13, wherein the radical generating catalyst has a Lewis acidity of no lower than 0.4 eV

**15.** The epithelial cancer therapeutic agent according to any one of claims 1 to 14, which is an epithelial cancer cell apoptosis promoting agent.

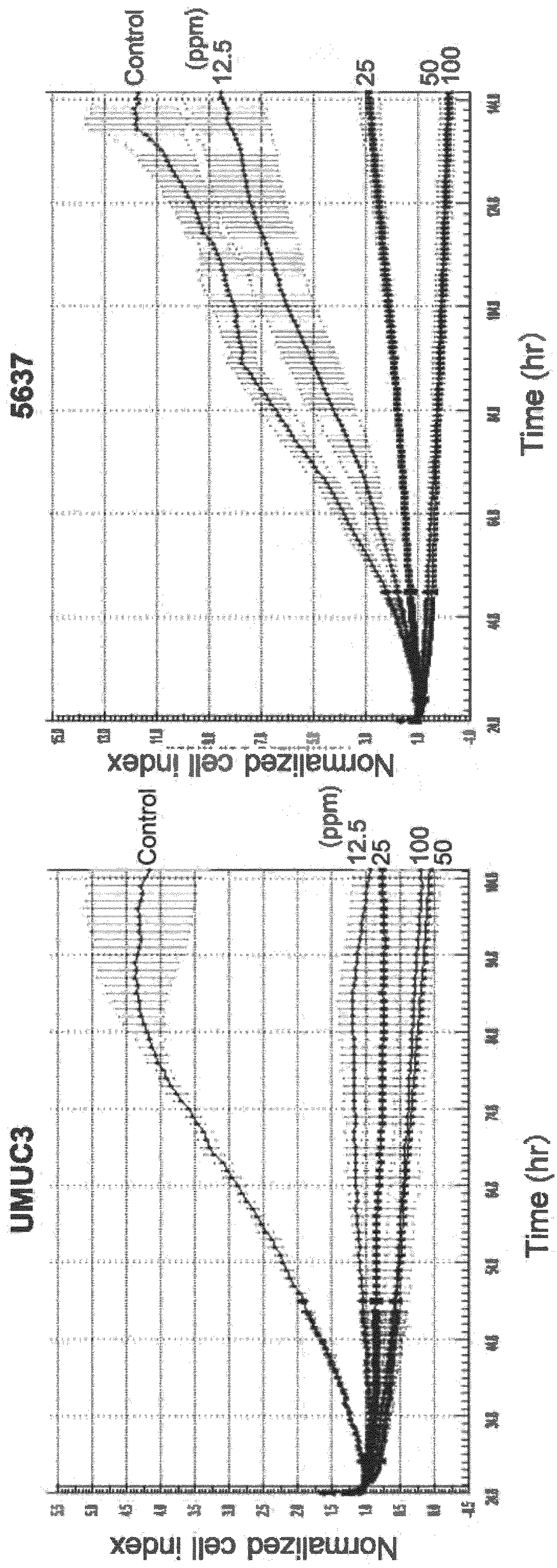
UMUC3
Normalized cell index
Time (hr)
Control
(ppm)
12.5
25
100
50
5637
Normalized cell index
Time (hr)
Control
(ppm)
12.5
25
50
100

FIG. 1

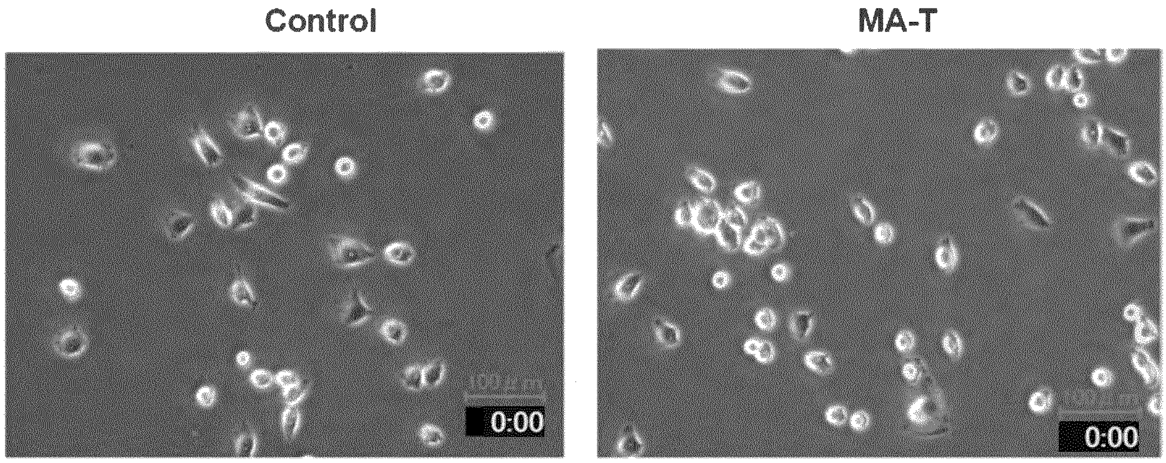


FIG. 2

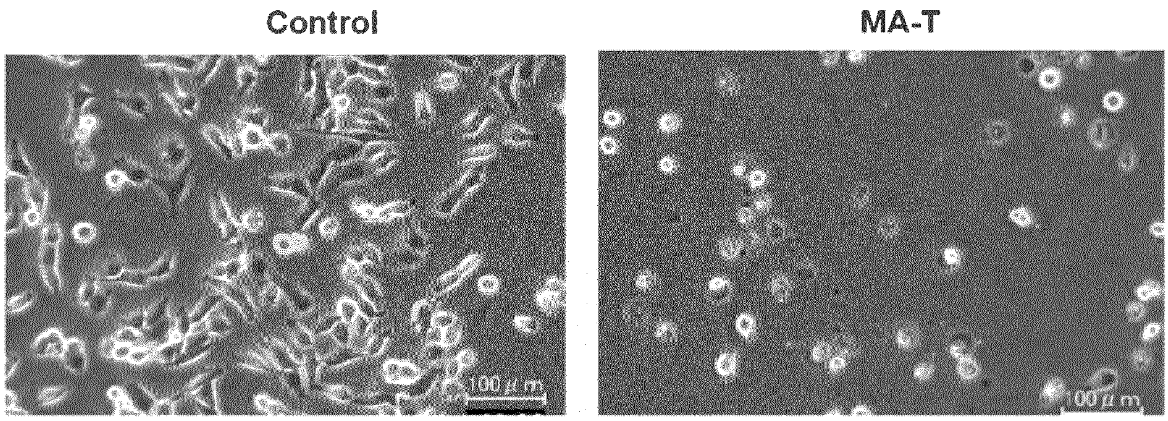


FIG. 3

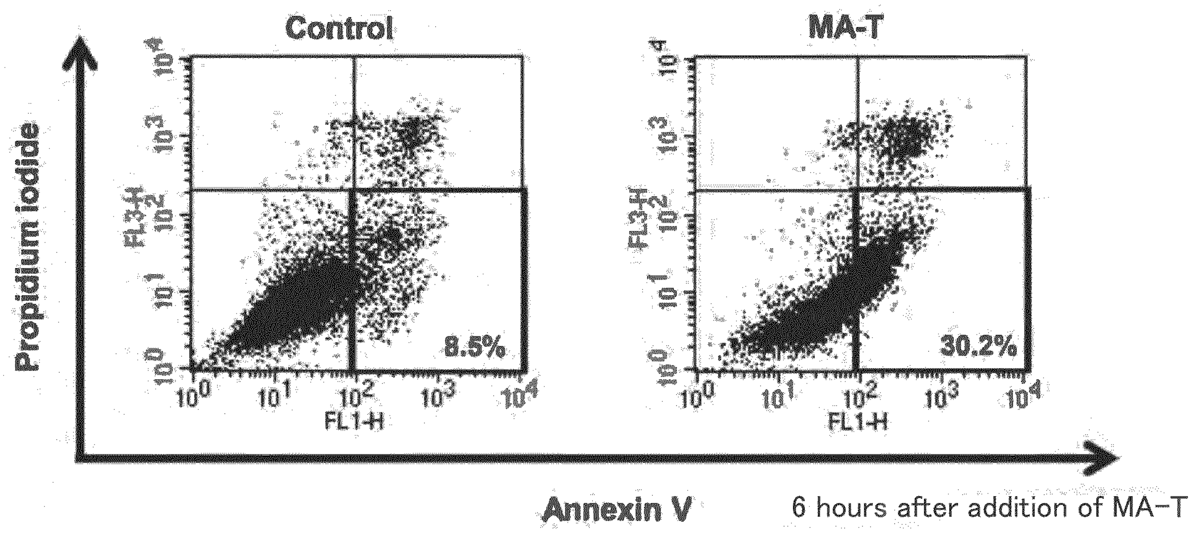


FIG. 4

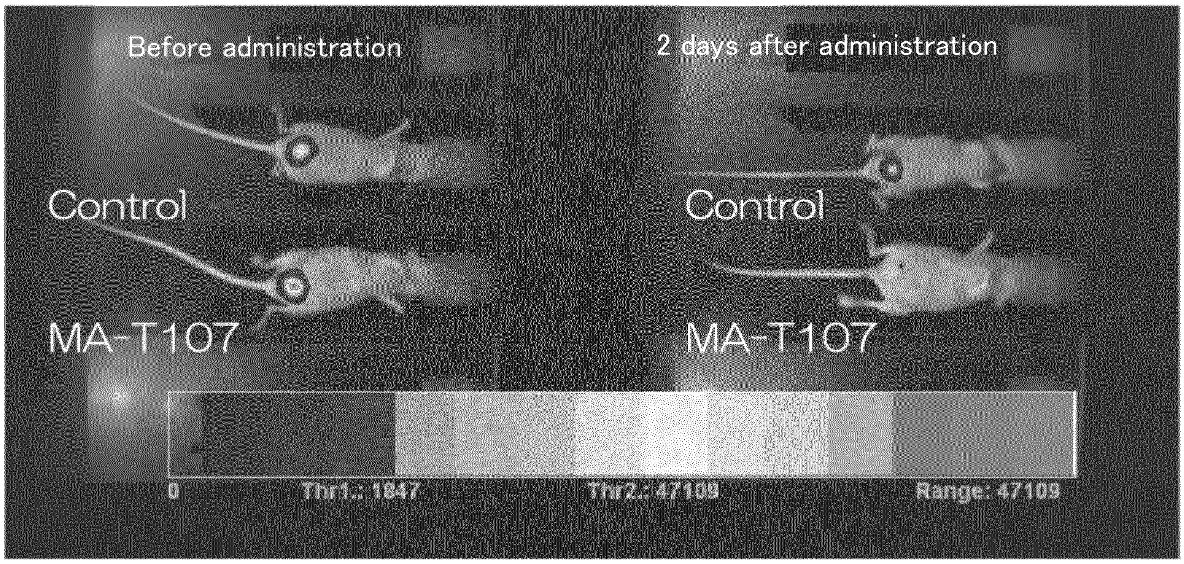


FIG. 5

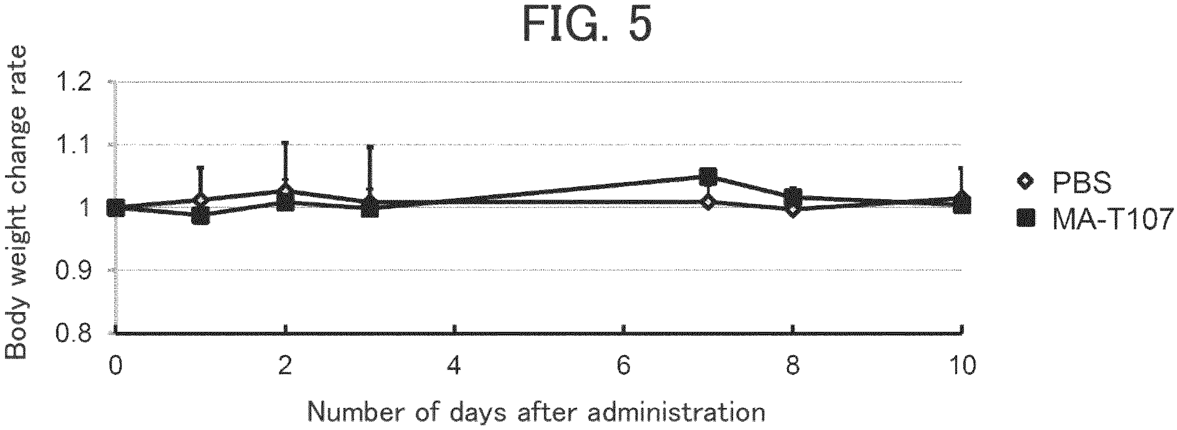


FIG. 6

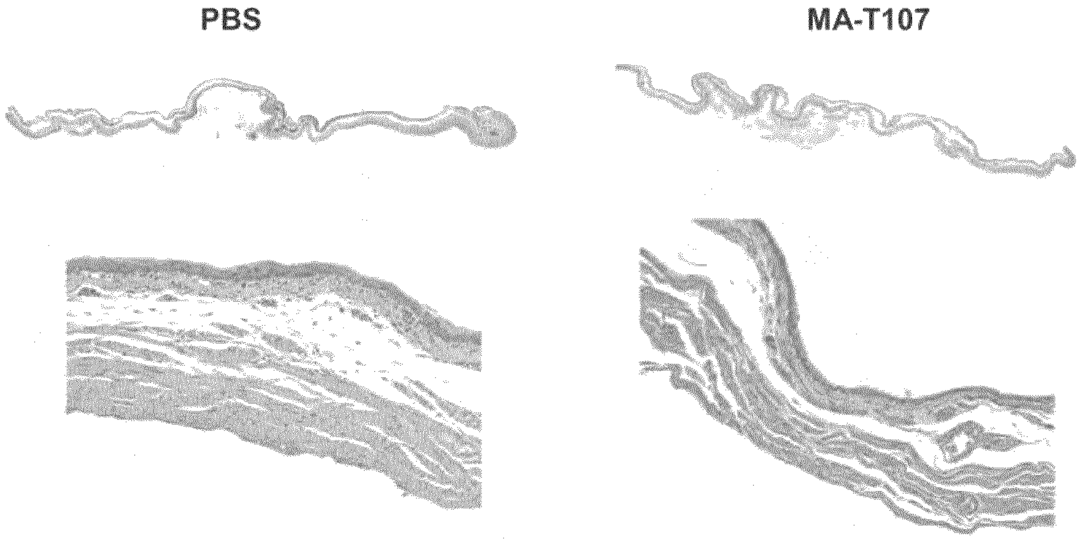


FIG. 7

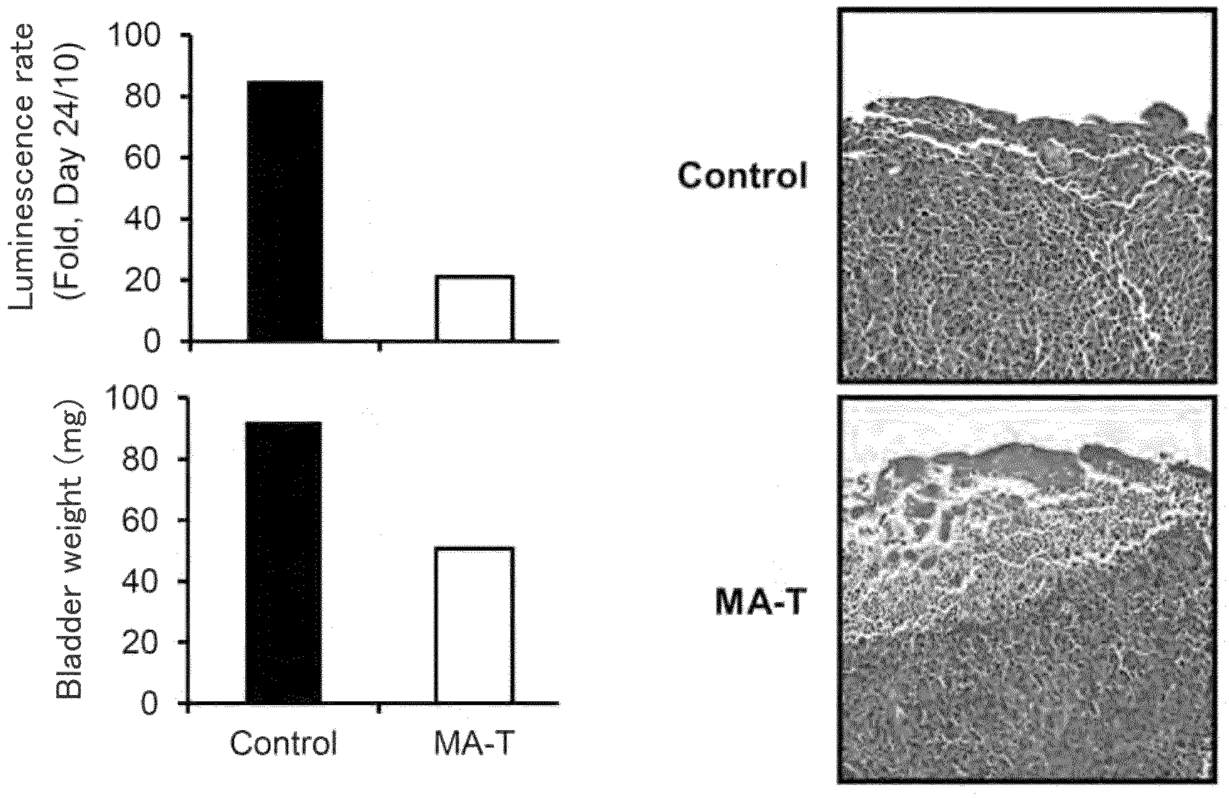


FIG. 8

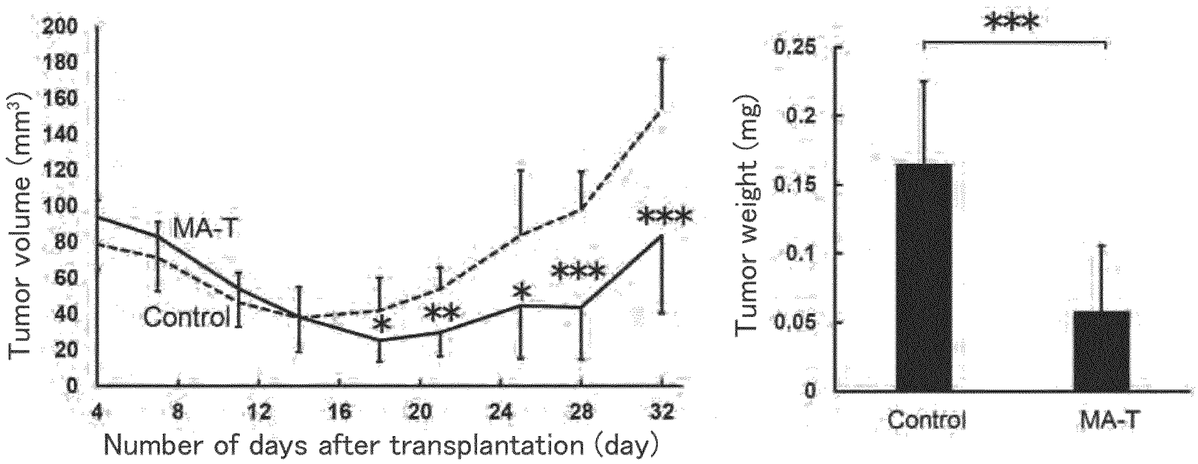


BLC115: 69 years old, male, pT1, Grade 2 (High), initial onset

Data represent mean±S.D. ( n=8). *P<0.05, **P<0.01, ***P<0.001.

FIG. 9

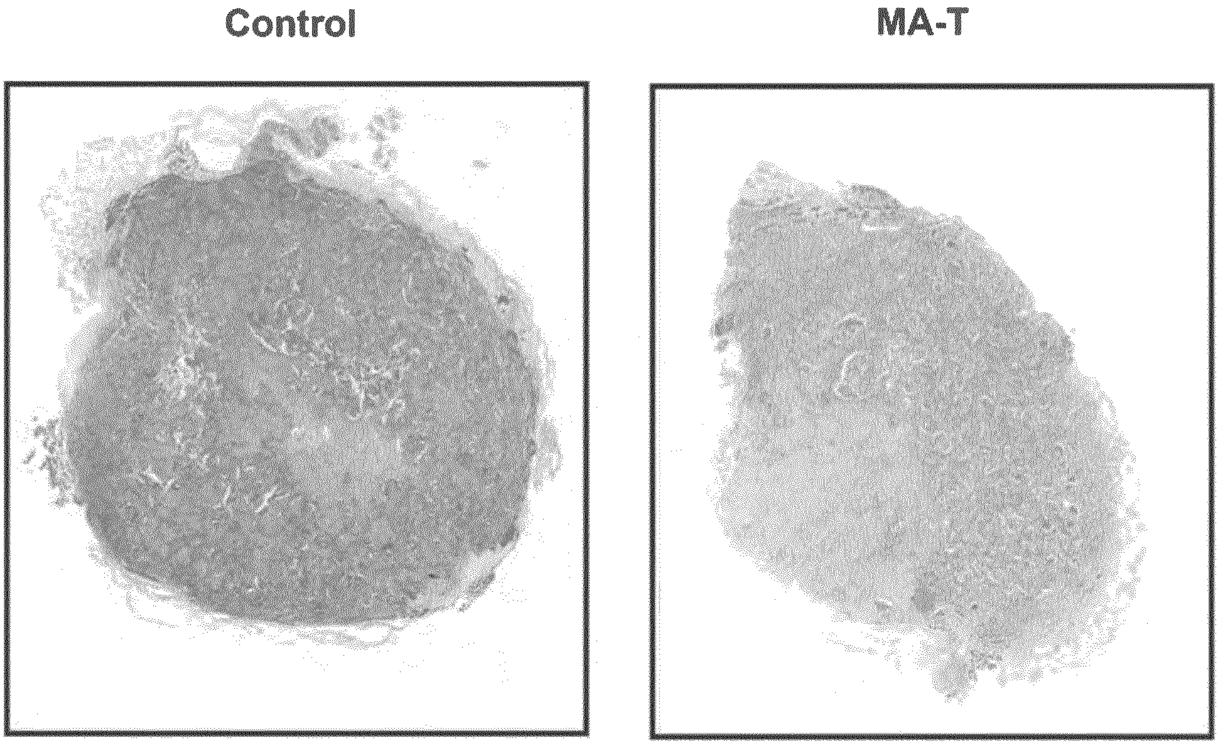
Control
MA-T

FIG. 10

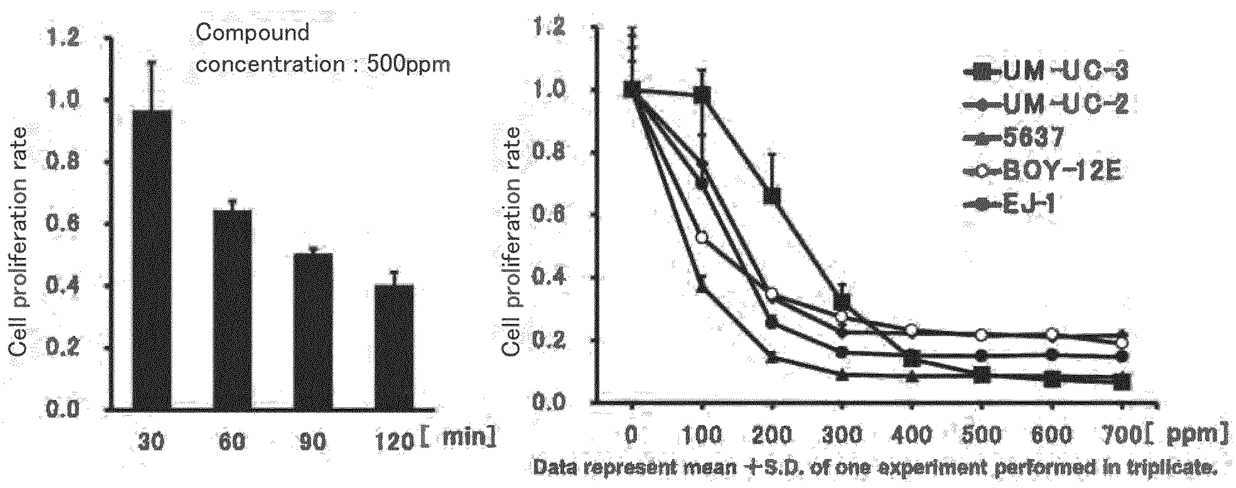
Compound concentration : 500ppm
Cell proliferation rate
1.2
1.0
0.8
0.6
0.4
0.2
0.0
30
60
90
120 [ min]
Cell proliferation rate
UM-UC-3
UM-UC-2
5637
BOY-12E
EJ-1
0
100
200
300
400
500
600
700[ ppm]
Data represent mean +S.D. of one experiment performed in triplicate.

FIG. 11

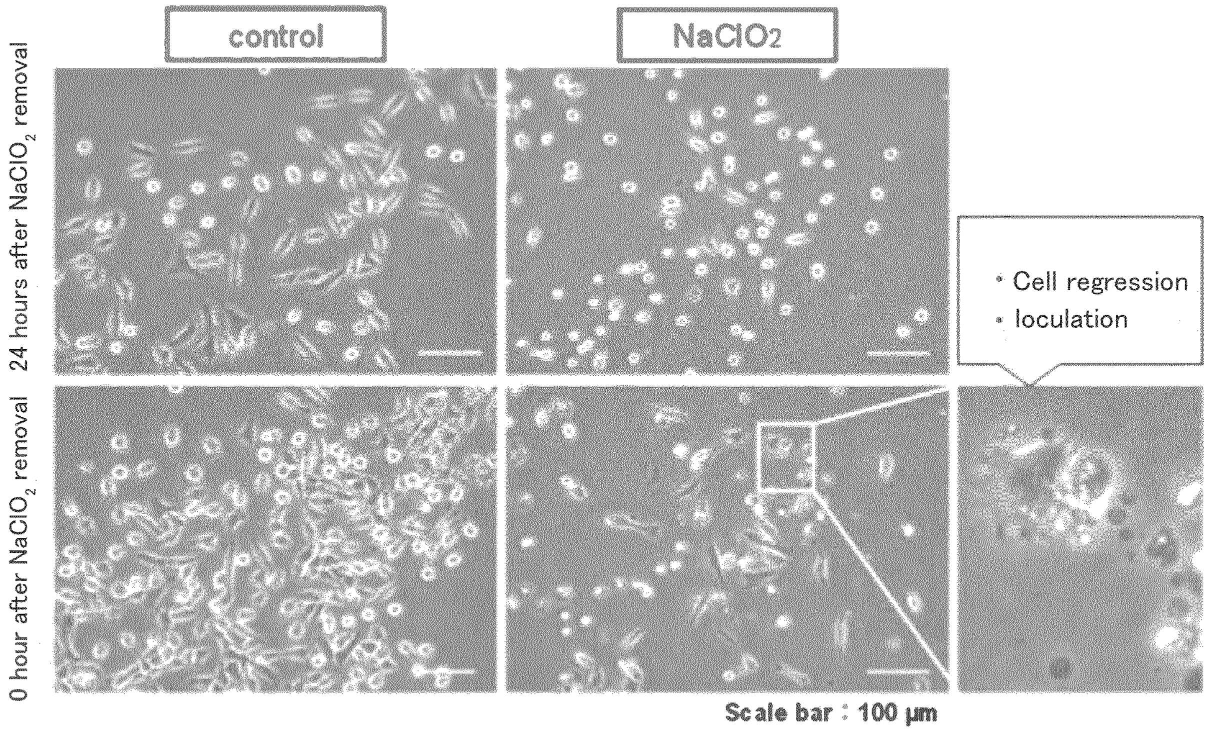
control
NaClO2
24 hours after $NaClO_2$ removal
0 hour after $NaClO_2$ removal
• Cell regression
• loculation
Scale bar : 100 μm

FIG. 12

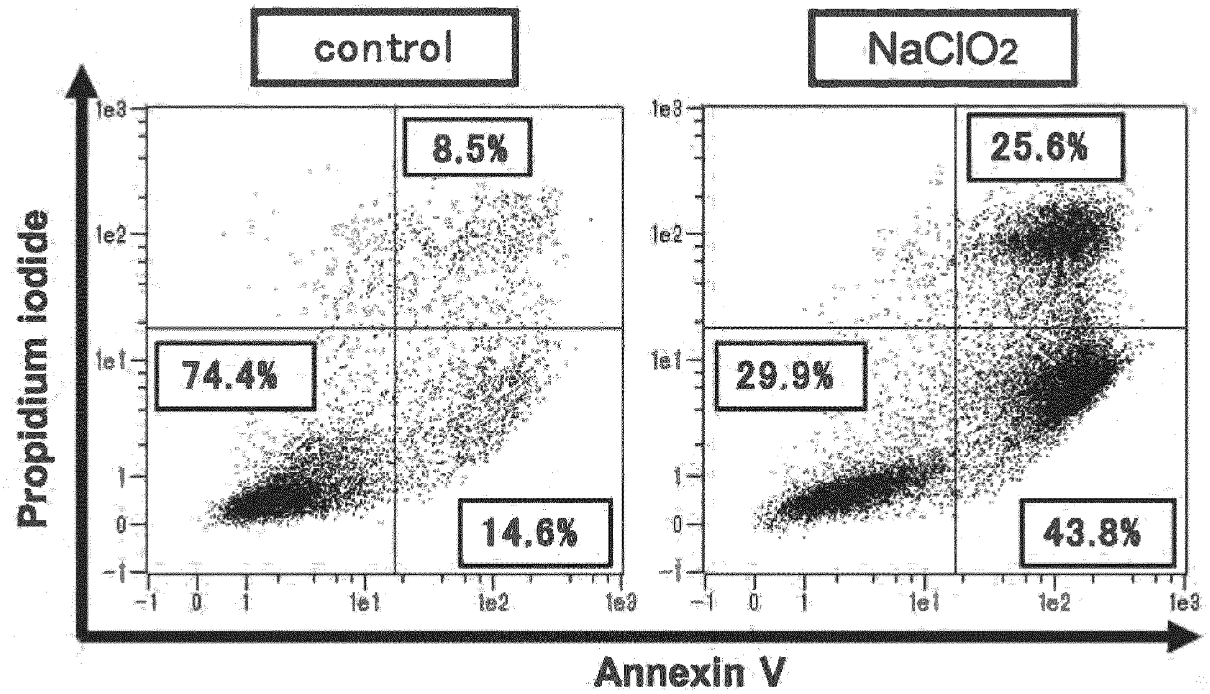
control
NaClO2
Propidium iodide
Annexin V
8.5%
74.4%
14.6%
25.6%
29.9%
43.8%
1e3
1e2
1e1
1
0
-1
1e1
1e2
1e3

FIG. 13

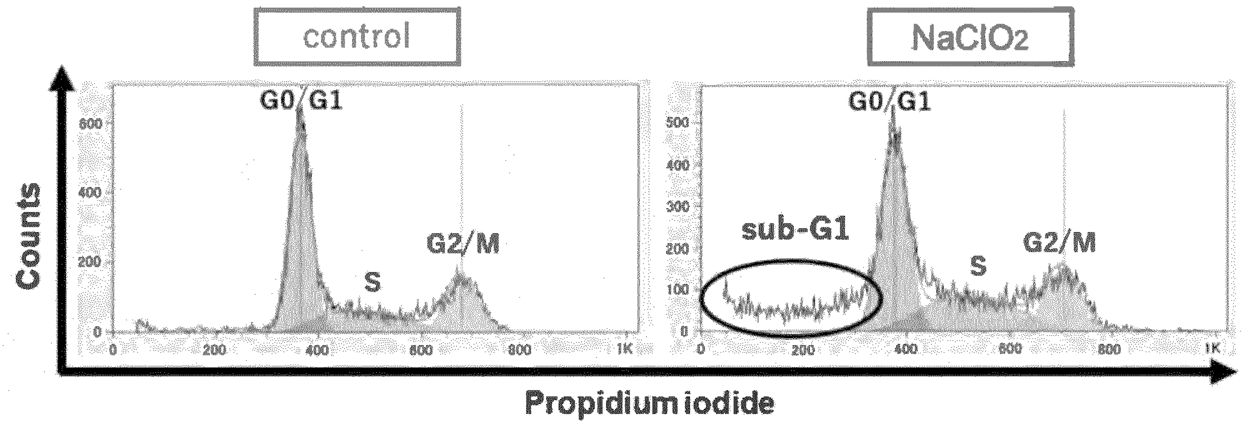
control
NaClO2
Counts
G0/G1
S
G2/M
sub-G1
0
200
400
600
800
1K
500
300
100
Propidium iodide

FIG. 14

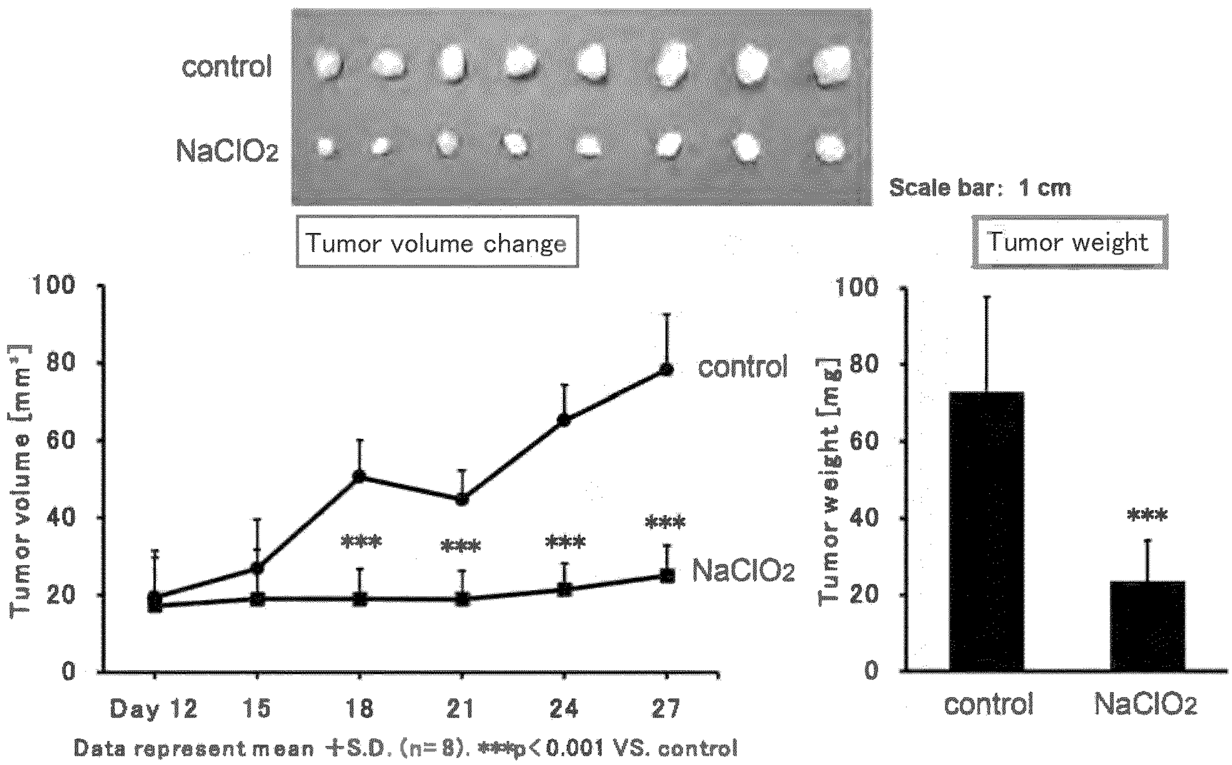
control
NaClO2
Scale bar: 1 cm
Tumor volume change
Tumor weight
Tumor volume [mm³]
Tumor weight [mg]
100
80
60
40
20
0
***
Day 12
15
18
21
24
27
Data represent mean +S.D. (n=8). ***p<0.001 VS. control

FIG. 15

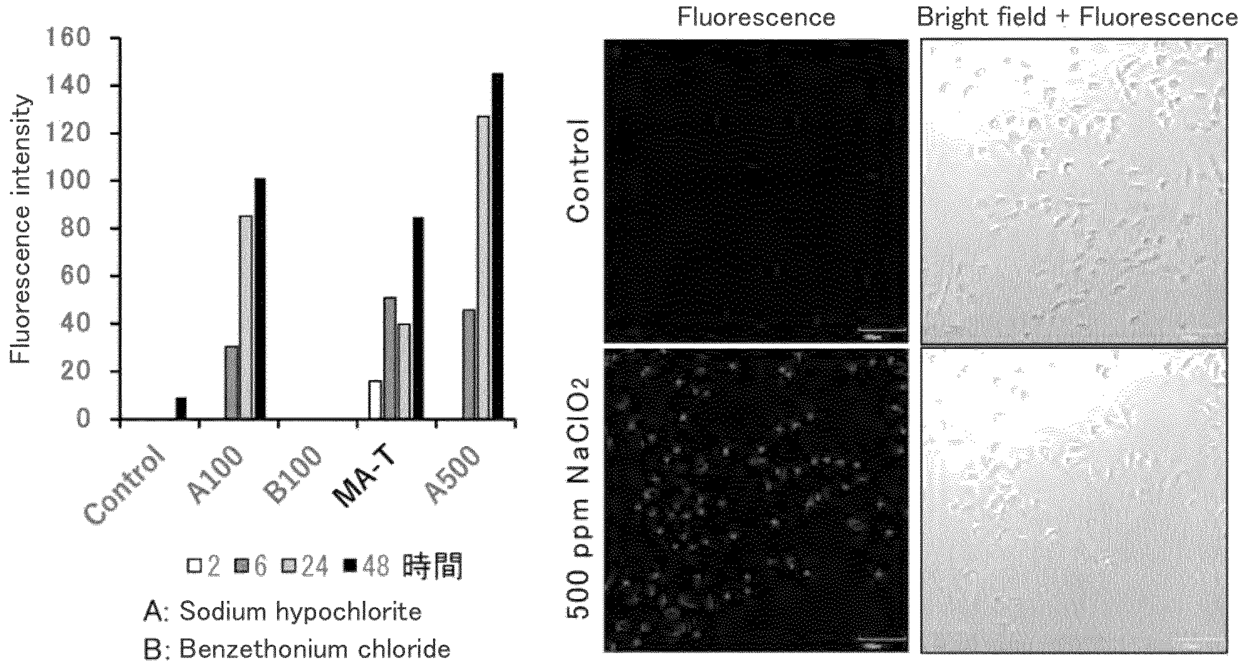
Fluorescence intensity
160
140
120
100
80
60
40
20
0
Control
A100
B100
MA-T
A500
□2 ■6 ■24 ■48 時間
A: Sodium hypochlorite
B: Benzethonium chloride
Fluorescence
Bright field + Fluorescence
Control
500 ppm NaClO2

FIG. 16

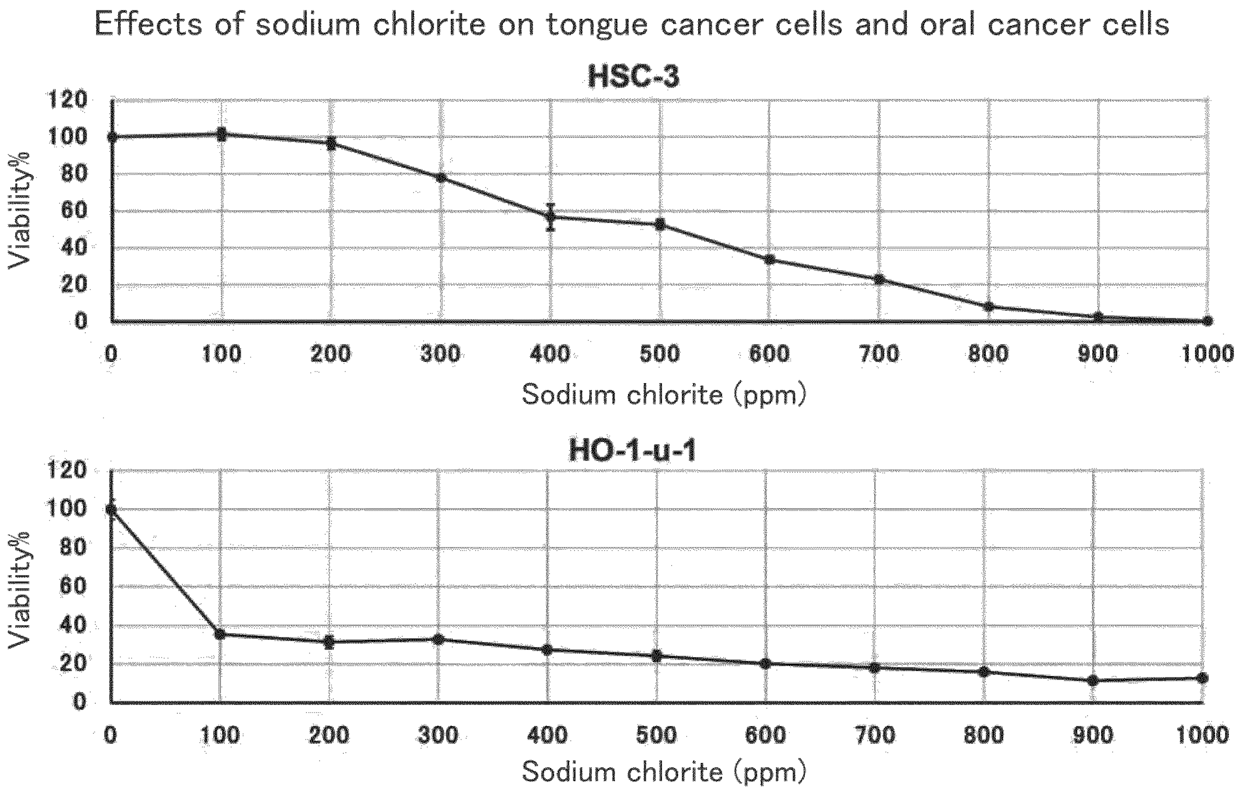
Effects of sodium chlorite on tongue cancer cells and oral cancer cells
HSC-3
Viability%
120
100
80
60
40
20
0
0 100 200 300 400 500 600 700 800 900 1000
Sodium chlorite (ppm)
HO-1-u-1
Viability%
120
100
80
60
40
20
0
0 100 200 300 400 500 600 700 800 900 1000
Sodium chlorite (ppm)

FIG. 17

Effects of sodium chlorite on uterine cancer cells and cervical cancer cells

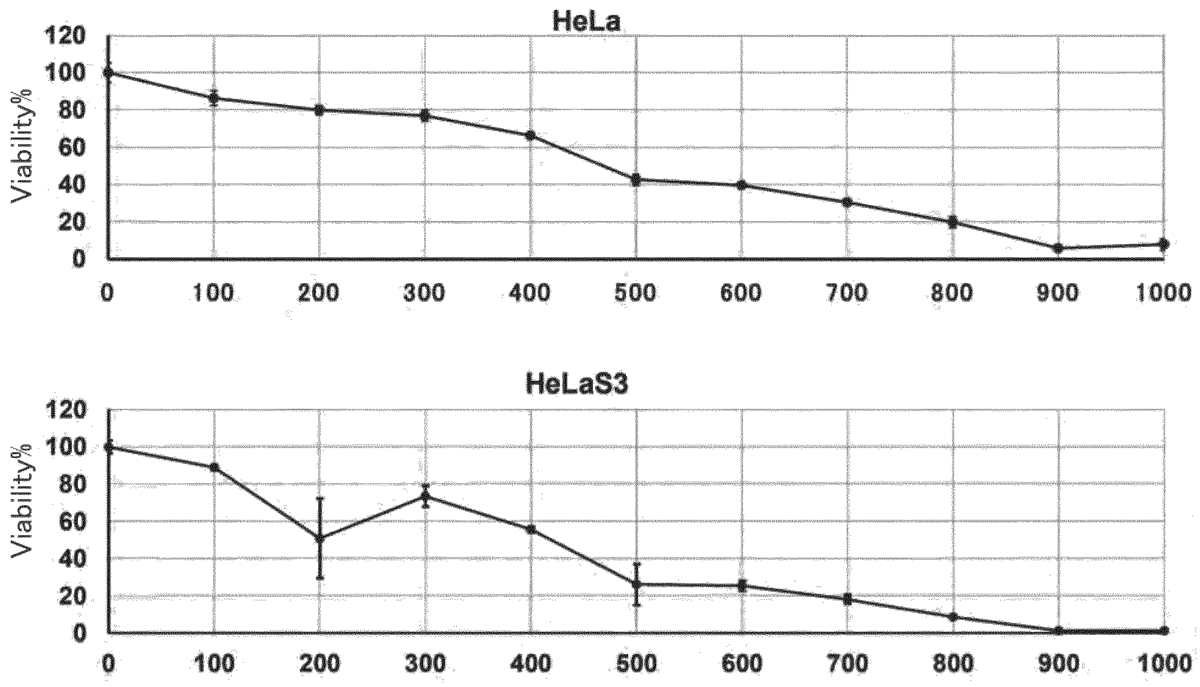


FIG. 18

Effects of sodium chlorite on colon cancer cells

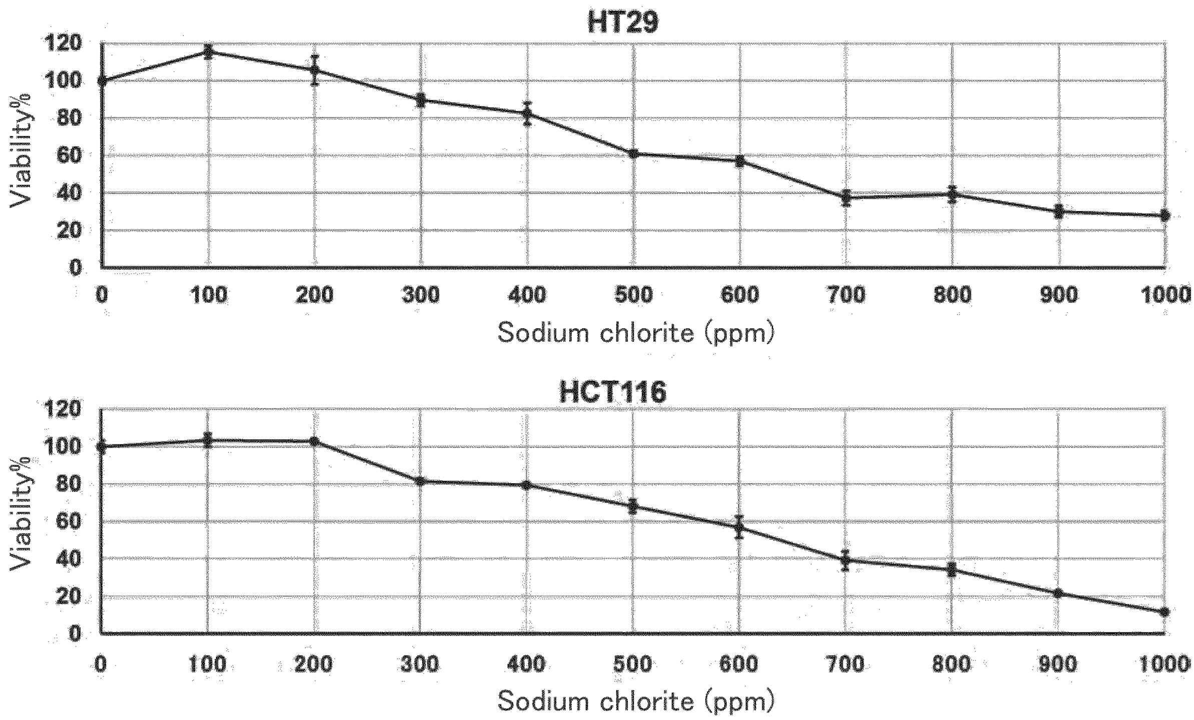


FIG. 19

Study on combined effect of agent A and agent B on uterine cancer cell HeLa

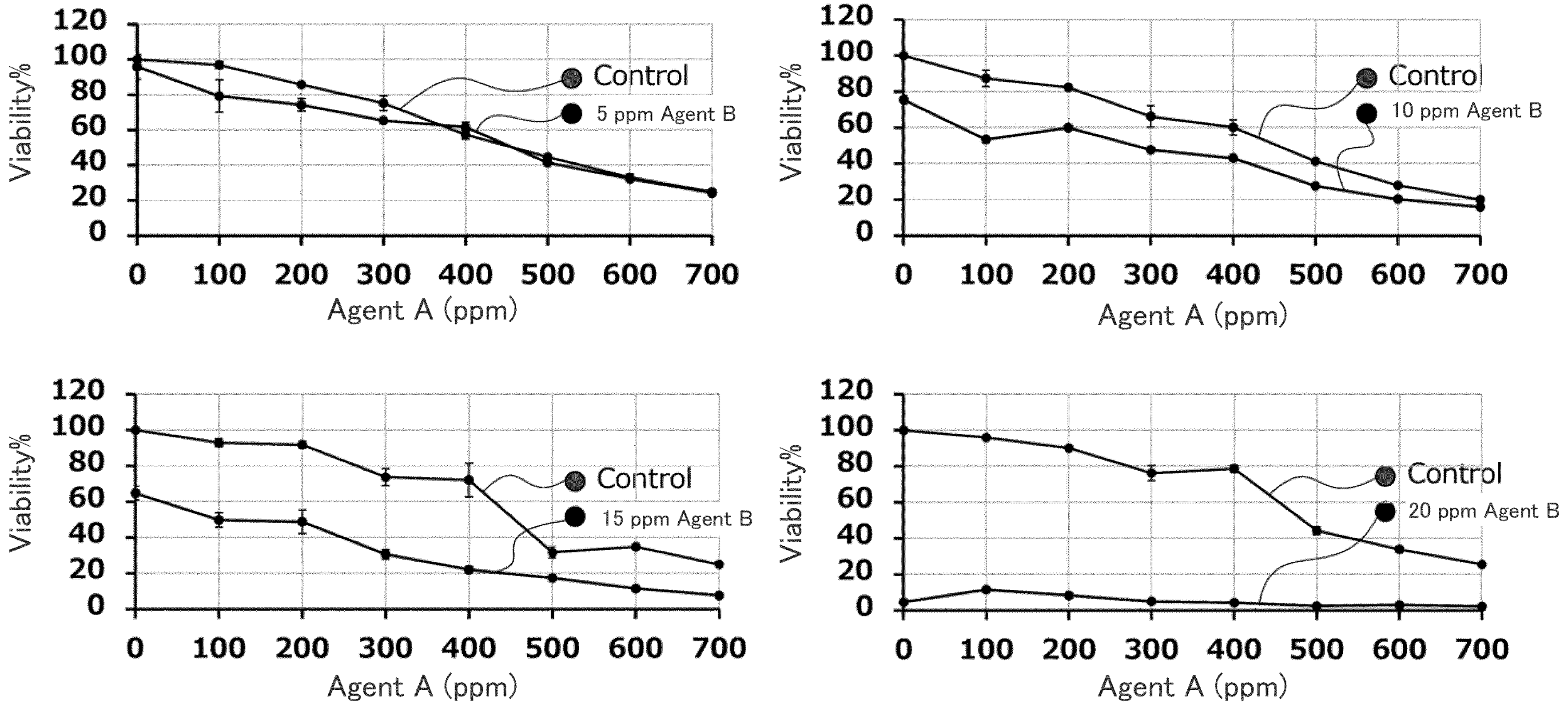


Bladder cancer cell proliferation assay results 48 hours after incubation with agent A and agent B for 1 hour
Mean ± SD of triplicate experiments

FIG. 20

Study on combined effect of agent A and agent B on cervical cancer cell HeLaS3

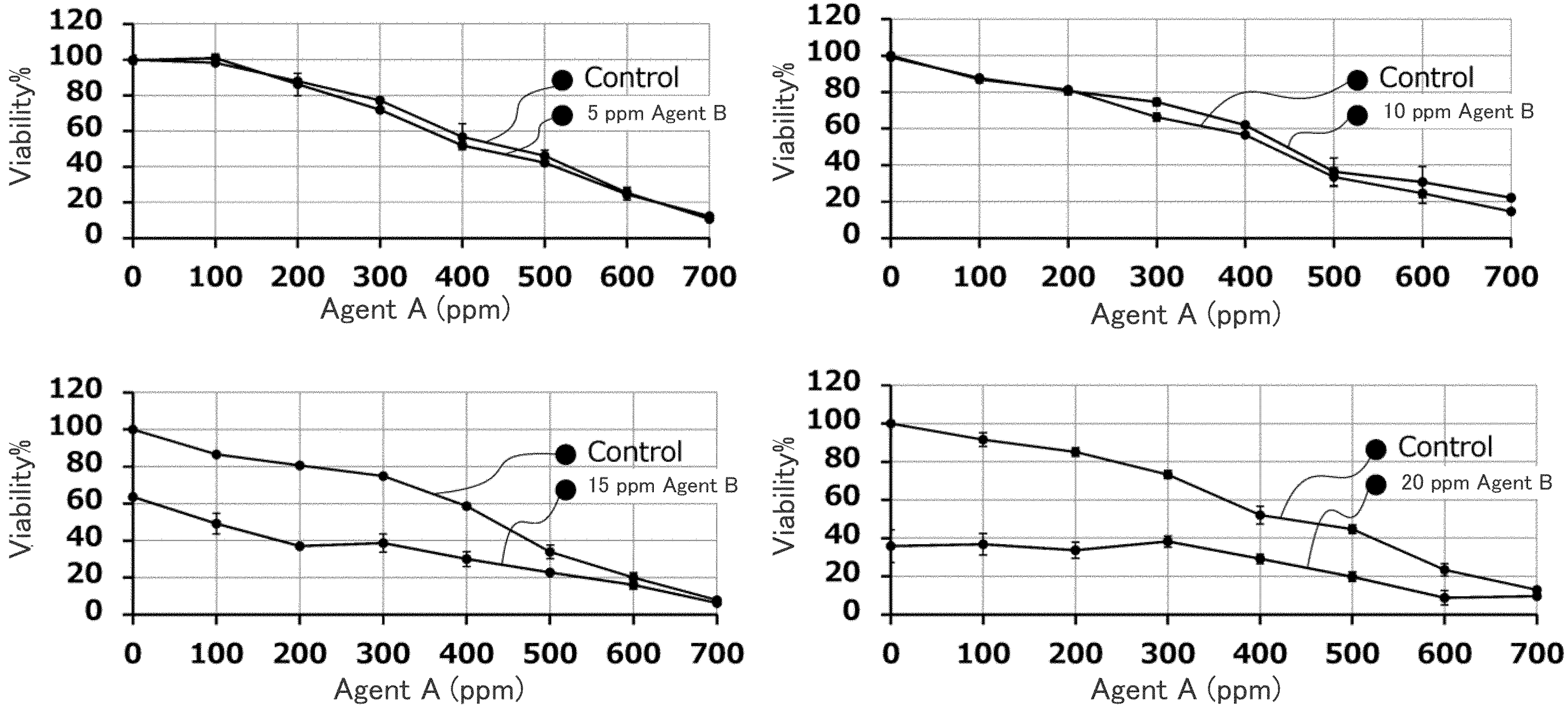


Bladder cancer cell proliferation assay results 48 hours after incubation with agent A and agent B for 1 hour

Mean ± SD of triplicate experiments

FIG. 21

Effects of sodium chlorite on pancreatic cancer primary culture cells

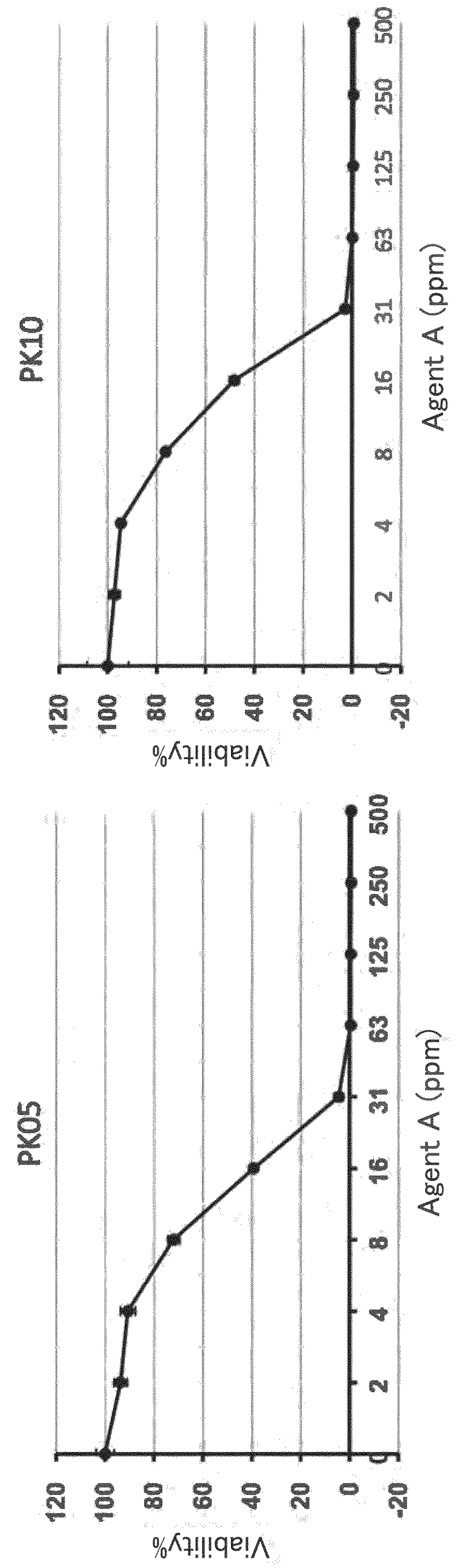


FIG. 22

## INTERNATIONAL SEARCH REPORT

International application No. PCT/JP2021/019079

**A. CLASSIFICATION OF SUBJECT MATTER**

A61K 33/40(2006.01)i; A61K 31/14(2006.01)i; A61P 35/00(2006.01)i; A61P 43/00(2006.01)i
FI: A61K33/40; A61P35/00; A61P43/00 105; A61K31/14

According to International Patent Classification (IPC) or to both national classification and IPC

**B. FIELDS SEARCHED**

Minimum documentation searched (classification system followed by classification symbols)
A61K33/40; A61K31/14; A61P35/00; A61P43/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| X<br>Y | US 2019/0000875 A1 (ALLIGER, Howard) 03 January 2019 (2019-01-03) claims, paragraphs [0003]-[0005], [0078], example 1, experimental FPI-1, FPI-1A, etc. | 1-8, 15<br>2, 3, 9-14 |
| Y | KR 10-2016-0145434 A (KOREA UNIVERSITY RESEARCH AND BUSINESS FOUNDATION) 20 December 2016 (2016-12-20) claims, paragraphs [0013]-[0058], fig. 1-4 | 2, 3 |
| Y | WO 2017/104797 A1 (ACENET INC.) 22 June 2017 (2017-06-22) claims, paragraph [0011], examples, etc. | 9-14 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>09 June 2021 (09.06.2021) | Date of mailing of the international search report<br>22 June 2021 (22.06.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**
Information on patent family members

International application No.
PCT/JP2021/019079

| Patent Documents referred in the Report | Publication Date | Patent Family | Publication Date |
|---|---|---|---|
| US 2019/0000875 A1 | 03 Jan. 2019 | US 10105389 B1 | |
| KR 10-2016-0145434 A | 20 Dec. 2016 | (Family: none) | |
| WO 2017/104797 A1 | 22 Jun. 2017 | US 2018/0369798 A1 claims, paragraph [0029], examples<br>EP 3391965 A1<br>CN 108430624 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020089253 A


### Non-patent literature cited in the description


- **HIROFUMI KOGA ; AKITO YAMAGUCHI.** *HINYOKI KEA Urological nursing,* 2014, vol. 19 (7), 731-736
- **OHKUBO, K. ; FUKUZUMI, S.** *Chem. Eur. J.,* 2000, vol. 6, 4532
- *J. Am. Chem. Soc.,* 2002, vol. 124, 10270-10271
- *J. Org. Chem.,* 2003, vol. 68, 4720-4726